# EUROPEAN PATENT SPECIFICATION

(11) **EP 2 742 131 B1**
(45) Date of publication and mention of the grant of the patent: **28.11.2018**
(21) Application number: 12768896.8
(22) Date of filing: 07.08.2012
(51) Int. Cl.: C12N 9/00, C12N 9/10, C12N 5/10

(54) **METHODS AND MATERIALS FOR RECOMBINANT PRODUCTION OF SAFFRON COMPOUNDS**
VERFAHREN UND MATERIALIEN ZUR HERSTELLUNG REKOMBINANTER SAFRANVERBINDUNGEN
PROCÉDÉS ET MATIÈRES POUR LA PRODUCTION RECOMBINANTE DE COMPOSÉS DU SAFRAN

(30) Priority: 08.08.2011 US 201161521171 P; 16.12.2011 US 201161576460 P; 06.02.2012 US 201261595450 P
(43) Date of publication of application: 18.06.2014
(73) Proprietor: Evolva SA, 4153 Reinach (CH)
(72) Inventor: SONKAR, Shailendra, 600 041 Chennai (IN); KUMAR, Sathish, 603 106 Kancheepuram District (IN); PANCHAPAGESA, Murali, Taramani 600 113 Chennai (IN); HANSEN, Esben, Halkjaer, 1972 Frederiksberg (DK); HANSEN, Klavs, Riishede, 2300 Copenhagen (DK); RAGHAVAN, Shriram, 600 061 Chennai (IN); HANSEN, Jorgen, 2000 Fredericksburg (DK); KUMAR, Kalyan K., 600 096 Chennai (IN)
(74) Representative: Rees, Kerry
(86) International application number: PCT/IB2012/001513
(87) International publication number: WO 2013/021261

(56) References cited:
- QIU DONGLIANG ET AL: "Improved protocol for Agrobacterium mediated transformation of tomato and production of transgenic plants containing carotenoid biosynthetic gene CsZCD", SCIENTIA HORTICULTURAE (AMSTERDAM), vol. 112, no. 2, March 2007 (2007-03), pages 172-175, XP002687286, ISSN: 0304-4238
- ANGELA RUBIO MORAGA ET AL: "Glucosylation of the saffron apocarotenoid crocetin by a glucosyltransferase isolated from Crocus sativus stigmas", PLANTA, vol. 219, no. 6, 1 October 2004 (2004-10-01), pages 955-966, XP055044069, ISSN: 0032-0935, DOI: 10.1007/s00425-004-1299-1
- BOUVIER F ET AL: "Oxidative remodeling of chromoplast carotenoids: Identification of the carotenoid dioxygenase CsCCD and CsZCD genes involved in Crocus secondary metabolite biogenesis", THE PLANT CELL, AMERICAN SOCIETY OF PLANT BIOLOGISTS, US, vol. 15, no. 1, 1 January 2003 (2003-01-01), pages 47-62, XP002381214, ISSN: 1040-4651, DOI: 10.1105/TPC.006536
- SANDMANN G ET AL: "Understanding carotenoid metabolism as a necessity for genetic engineering of crop plants", METABOLIC ENGINEERING, ACADEMIC PRESS, US, vol. 8, no. 4, 1 July 2006 (2006-07-01), pages 291-302, XP024946934, ISSN: 1096-7176, DOI: 10.1016/J.YMBEN.2006.01.005 [retrieved on 2006-07-01]
- Brian Basco ET AL: "Saffron in a Kan: Production of Safranal, Picrocin and Croncin", , 1 January 2012 (2012-01-01), XP055044417, Retrieved from the Internet: URL:http://2012.igem.org/files/poster/Wash U.pdf [retrieved on 2012-11-15]
- Qiu Dongliang: "Improved protocol for Agrobacterium mediated transformation of tomato and production of transgenic plants containing carotenoid biosynthetic gene CsZCD", Scientia Horticulturae, 1 January 2007 (2007-01-01), pages 172-175, XP055044077, Retrieved from the Internet: URL:http://ac.els-cdn.com/S030442380600500 0/1-s2.0-S0304423806005000-main.pdf?_tid=c 6c44a08-2da2-11e2-852e-00000aab0f26&acdnat =1352818971_2253e6630db6348d6648e4859bb2fb 21 [retrieved on 2012-11-13]

## Description

This application claims priority from US Provisional Application No. 61/ 521171 filed on August 8, 2011, US Provisional Application No. 61/576460 filed on December 16, 2011, and US Provisional Application No. 61/595450 filed on February 6, 2012.

### TECHNICAL FIELD

This invention relates to material for recombinantly producing compounds from *Crocus sativus,* the saffron plant, and more particularly to materials for recombinantly producing flavorant, aromatant, and colorant compounds from the saffron plant in a recombinant host,

### BACKGROUND

Saffron is a dried spice prepared by extraction from the stigmas of the *Crocus sativus* L. flower, and is thought to have been used for over 3500 years. This spice has been used historically for numerous medicinal purposes, but in recent times is largely utilized for its colorant properties. Crocetin, one of the major components of saffron, has antioxidant properties similar to related carotenoid-type molecules, as well as being a colorant. The main pigment of saffron is crocin, which is a mixture of glycosides that impart yellowish red colors. A major constituent of crocin is α-crocin, which is yellow in color. Safranal is thought to be a product of the drying process and has odorant qualities as well, that can be utilized in food preparation. Safranal is the aglycone form of the bitter part of the saffron extracts, picrocrocin, which is colorless. Thus, saffron extracts are used for many purposes, as a colorant or a flavorant, or for its odorant properties.

The saffron plant is grown commercially in many countries including Italy, France, India, Spain, Greece, Morocco, Turkey, Switzerland, Israel, Pakistan, Azerbaijan, China, Egypt, United Arab Emirates, Japan, Australia, and Iran. Iran produces approximately 80% of the total world annual saffron production (estimated to be just over 200 tons). It has been reported that over 150,000 flowers are required for 1 kg of product. Plant breeding efforts to increase yields are complicated by the triploidy of the plant's genome, resulting in sterile plants. In addition, the plant is in bloom only for about 15 days starting in middle or late October. Typically, production involves manual removal of the stigmas from the flower which is also an inefficient process. Selling prices of over $1000/kg of saffron are typical. An attractive alternative is bio-conversion or *de novo* biosynthesis of the components of saffron.

"Glucosylation of the Saffron Apocarotenoid Crocetin by a Glucosyltransferase Isolated from Crocus Sativus Stigmas", Moraga et al., Planta vol. 219, no. 6, 1 October 2004, pages 955 - 966 suggests that three major apocarotenoids- crocin, crocetin and picrocrocin- are responsible for the colour and bitter taste of saffron. It further states that the final step in the biosynthesis of the 20-carbon esterified carotenoid crocin is the transformation of the insoluble crocetin into a soluble and stable storage form by glucosylation. These glucosylation reactions are catalysed by glucosyltransferases (GTases) that play a crucial role in natural-product biosynthesis. Using degenerate primers designed to match the plant secondary product GTase (PSPG) box, the cloning of two cDNAs, UGTCs2 and UGTCs3, from C. sativus stigmas that encode putative polypeptides of 460 and 475 amino acids, respectively is described.

The authors state that these genes were expressed differentially in saffron tissues. UGTCs2 was mainly expressed in fully developed stigmas, whereas UGTCs3 was mainly expressed in stamens. The UGTCs2 transcript was not detected in the stigma tissue of a Crocus species that does not synthesize crocin, while UGTCs3 and other structural genes for carotenoid biosynthesis were expressed in the stigma of all tested Crocus species. To identify the biochemical function of UGTCs2, the isolated cDNA was expressed in Escherichia coli cells. The recombinant protein UGTCs2 had glucosylation activity against crocetin, crocetin beta-D-glucosyl ester and crocetin beta-D-gentibiosyl ester.

### SUMMARY

Thus, according to the present invention there is provided a recombinant, carotenoid producing host cell comprising:
(a) an exogenous nucleic acid encoding a zeaxanthin cleavage dioxygenase (ZCD), wherein said ZCD is optionally a *Crocus sativus* ZCD; and
(b) an exogenous nucleic acid encoding a β-carotene hydroxylase (CH),
wherein the host cell produces detectable amounts of any one or more of crocetin dialdehyde, crocetin, or hydroxyl-β-cyclocitral (HBC).

This disclosure is based on the discovery of methods and materials for improving production of compounds from the saffron plant in recombinant hosts, as well as nucleotides and polypeptides useful in establishing the recombinant pathways for production of compounds such as picrocrocin, safranal, crocin, crocetin, or crocetin esters. This disclosure also relates to compositions containing crocetin and crocetin esters. The products may be produced singly and recombined for optimal characteristics in a food system or for medicinal supplements. In other embodiments the compounds may be produced as a mixture. In some embodiments, the host strain is a recombinant yeast. In other embodiments the nucleotides described herein may be used in plant genetics and to assist as markers in plant breeding strategies.

In one aspect, this document features a recombinant, carotenoid producing host (e.g., a microorganism) that includes an exogenous nucleic acid encoding a zeaxanthin cleavage dioxygenase (ZCD). The host can produce detectable amounts of crocetin and/or crocetin dialdehyde and/or Hydroxyl-β-cyclocitral (HBC). The ZCD can be a *Crocus sativus* ZCD.

The host can comprise endogenous genes encoding geranylgeranyl diphosphate synthase (GGPPS), a phytoene synthase, a phytoene dehydrogenase, and a β-carotene synthase.

The host further can comprise at least one exogenous nucleic acid encoding GGPPS, a phytoene synthase, a phytoene dehydrogenase, and a β-carotene synthase.

This document also features a recombinant host comprising at least one exogenous nucleic acid encoding a GGPPS, a phytoene synthase, a phytoene dehydrogenase, a β-carotene synthase, a β -carotene hydroxylase, and a zeaxanthin cleavage dioxygenase (ZCD) (e.g. a *Crocus sativus* ZCD). Expression of the at least one exogenous nucleic acid can produce detectable amounts of crocetin and/or crocetin dialdehyde in the host.

Any of the hosts described herein can further include an endogenous gene encoding an aldehyde dehydrogenase or an exogenous nucleic acid encoding an aldehyde dehydrogenase (ALD). The aldehyde dehydrogenase can be a *Saccharomyces cerevisiae* aldehyde dehydrogenase (e.g., ALD2-ALD6 or HFD1).

Any of the hosts described herein can further include an endogenous gene encoding a β -carotene hydroxylase or an exogenous nucleic acid encoding a β -carotene hydroxylase. The β-carotene hydroxylase can be a *Xanthophyllomyces dendrorhous* β - carotene hydroxylase.

Any of the hosts described herein further can include an exogenous nucleic acid encoding an aglycone O-glycosyl uridine 5'-diphospho (UDP) glycosyl transferase (O-glycosyl UGT). Such a host can produce detectable amounts of picrocrocin or crocin. The aglycone O-glycosyl UGT can be UGT85C2, UGT73-EV12, or a UGT71 hybrid enzyme. The aglycone O-glycosyl UGT also can be Cs VrUGT2 from *Crocus sativus.*

Any of the hosts described herein further can include an exogenous nucleic acid encoding an O-glycosyl UGT. Such a host can produce detectable amounts of crocetin mono and di glucosyl esters. The aglycone O-glycosyl UGT can be UGT76G1, or a UGT71 hybrid enzyme (e.g., 71C125571C2 and/or 71C125571E1).

Any of the hosts described herein further can include an exogenous nucleic acid encoding a UGT that catalyzes a β glucosyl linkage between two glucose moieties (e.g., a β 1,6 linkage). Such a host can produce a detectable amount of crocetin gentibiosyl ester. The UGT that catalyzes the β glucosyl linkage between two glucose moieties can be a UGT71 hybrid enzyme such as 71C125571C2 or 71C125571E1.

Any of the hosts described herein further can include an exogenous nucleic acid encoding a uridine-5'-diphosphoglucose (UDP-glucose)-crocetin 8,8'-glucosyltransferase. Such a host can produce a detectable amount of a crocetin monoglucoside. The UDP-glucose-crocetin 8,8'-glucosyltransferase can be a *Crocus* UDP-glucose-crocetin 8,8'-glucosyltransferase.

Any of the hosts described herein further can include an exogenous nucleic acid encoding a UGT that catalyzes a β glucosyl linkage between two glucose moieties (e.g., a β 1,6 linkage). Such a host can produce a detectable amount of crocin. The UGT that catalyzes the β glucosyl linkage between two glucose moieties can be UGT76G1, UN4522, or UN1671.

Any of the hosts described herein can be a microorganism, a plant, or a plant cell. The microorganism can be an oleaginous yeast, a Saccharomycete such as *Saccharomyces cerevisiae,* or *Escherichia coli.* The plant or plant cell can be *Crocus sativus.*

Any of the hosts described herein further can include an exogenous nucleic acid encoding one or more of deoxyxylulose 5-phosphate synthase (DXS), D-1-deoxyxylulose 5-phosphate reductoisomerase (DXR), 4-diphosphocytidyl-2-C-methyl-D-erythritol synthase (CMS), 4-diphosphocytidyl-2-C-methyl-D-erythritol kinase (CMK), 4-diphosphocytidyl-2-C-methyl-D-erythritol 2,4-cyclodiphosphate synthase (MCS), 1-hydroxy-2-methyl-2(E)-butenyl 4-diphosphate synthase (HDS), and 1-hydroxy-2-methyl-2(E)-butenyl 4-diphosphate reductase (HDR).

Any of the hosts described herein further can include an exogenous nucleic acid encoding one or more of truncated 3-hydroxy-3-methyl-glutaryl (HMG)-CoA reductase (tHMG), a mevalonate kinase (MK), a phosphomevalonate kinase (PMK), and a mevalonate pyrophosphate decarboxylase (MPPD).

In another aspect, this document features a method of producing picrocrocin. The method includes contacting HBC with an aglycone O-glycosyl UGT and UDP-glucose to produce picrocrocin, wherein the aglycone O-glycosyl UGT is selected from the group consisting of UGT85C2, UGT73-EV12, or a UGT71 hybrid enzyme. The UGT also can be Cs VrUGT2.

In yet another aspect, this document features an isolated nucleic acid encoding a UGT73 polypeptide. The UGT73 polypeptide can have at least 80% sequence identity to the UGT73 amino acid sequence set forth in FIG. 3. This document also features a nucleic acid construct comprising a regulatory region operably linked to such a nucleic acid as well as a recombinant host comprising such a nucleic acid or nucleic acid construct.

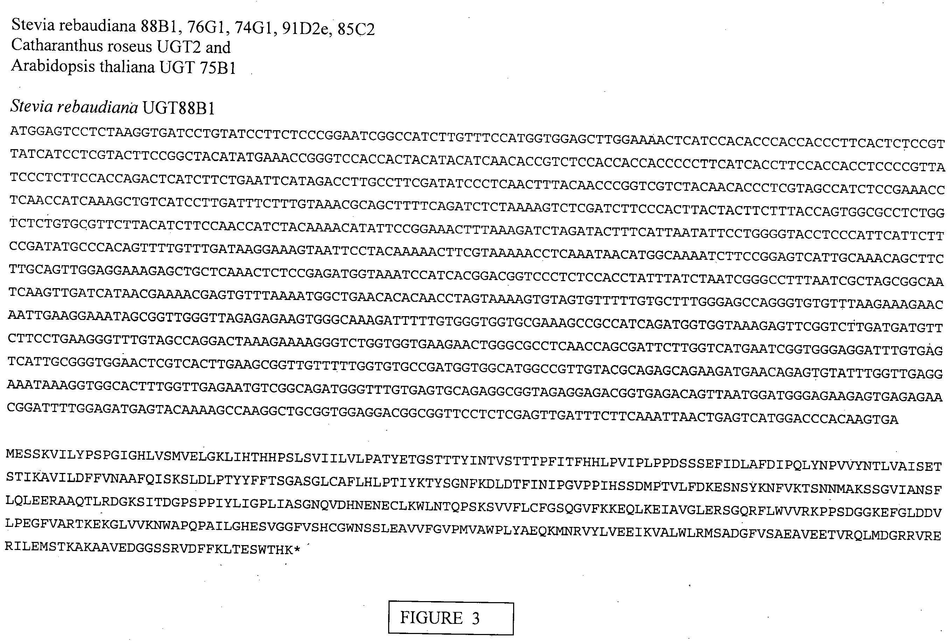

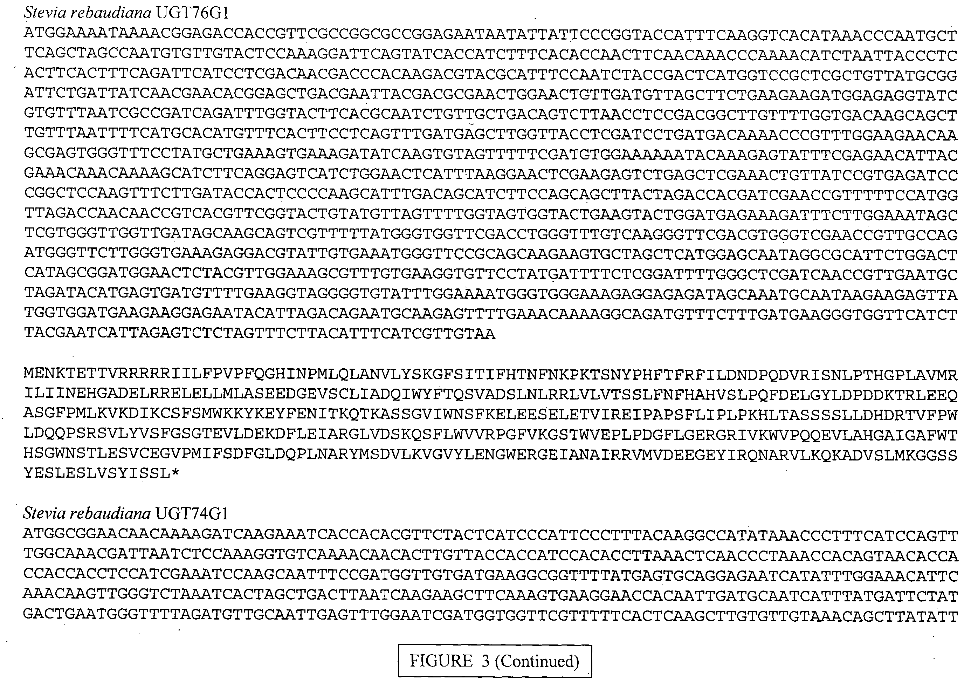

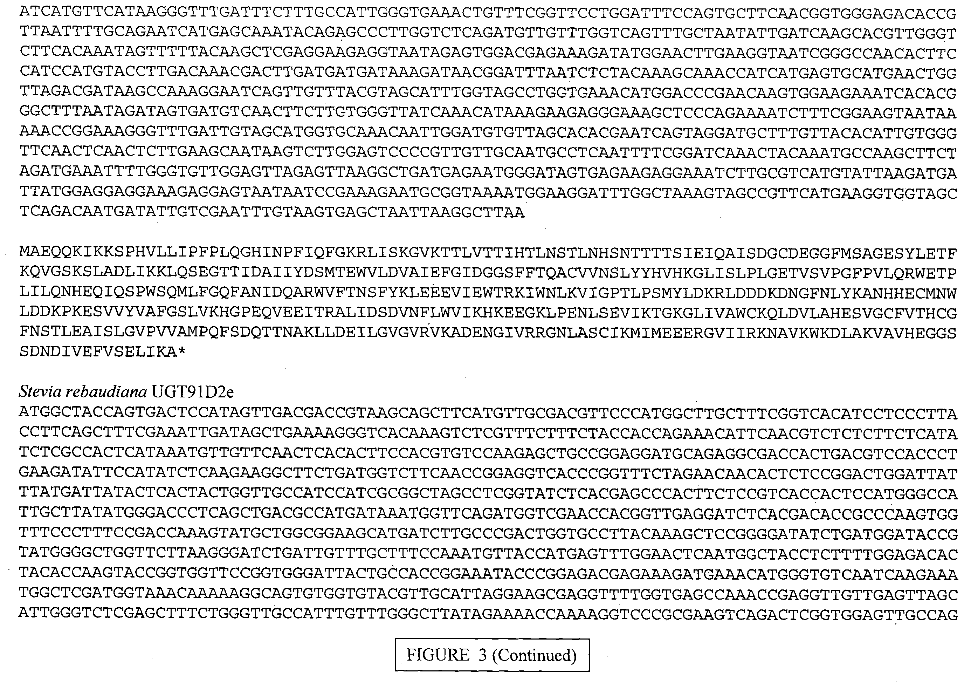

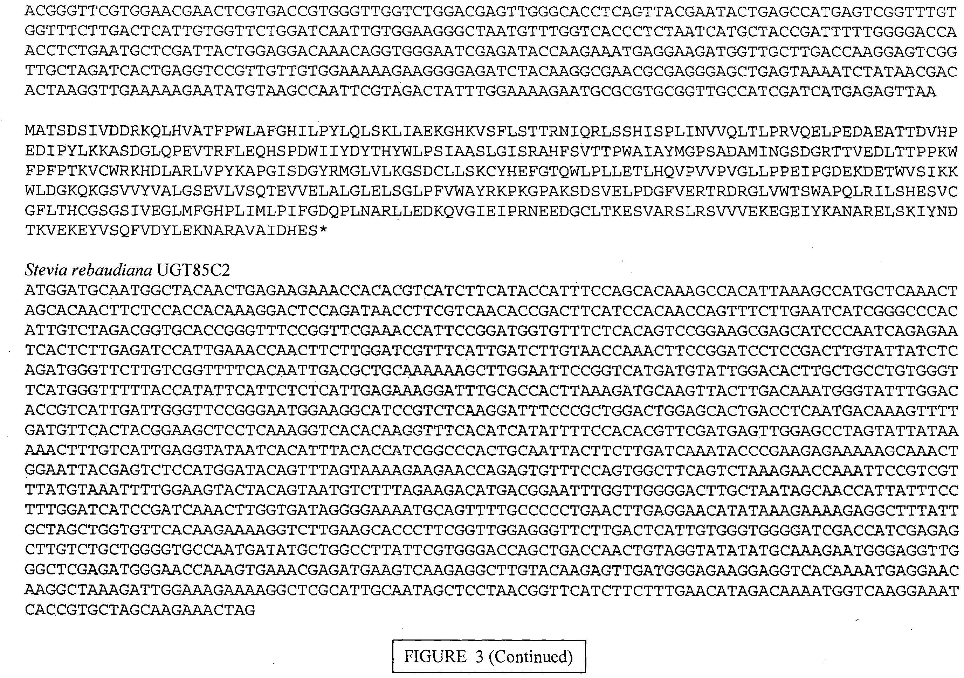

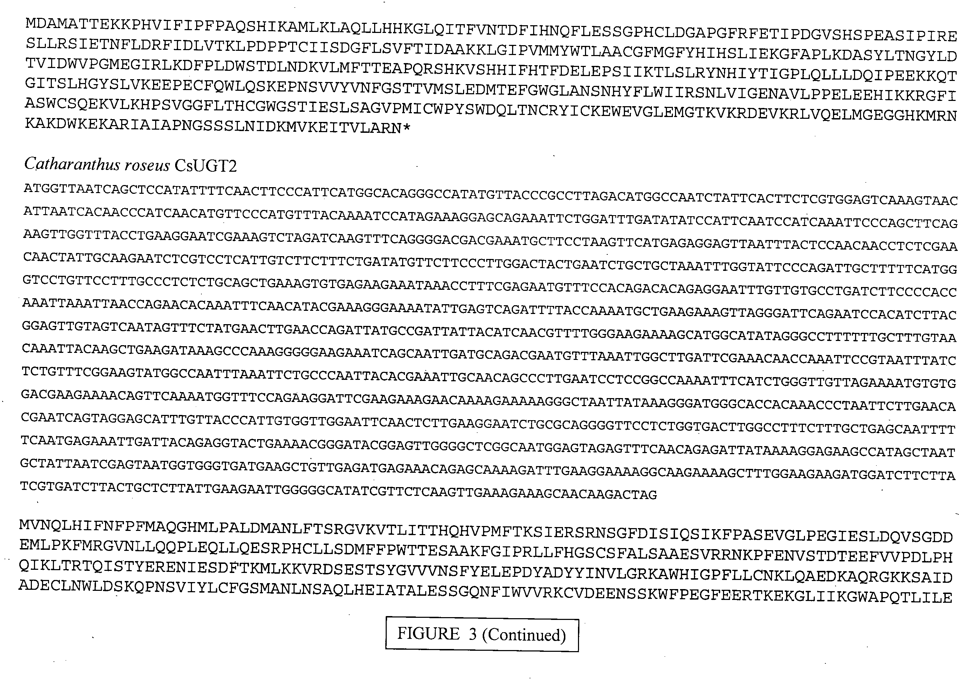

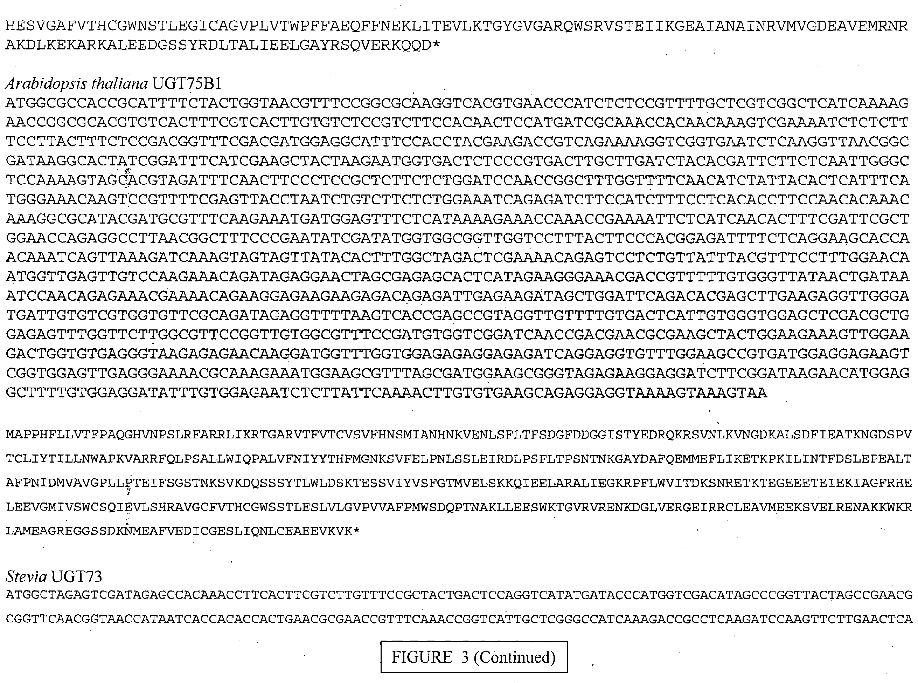

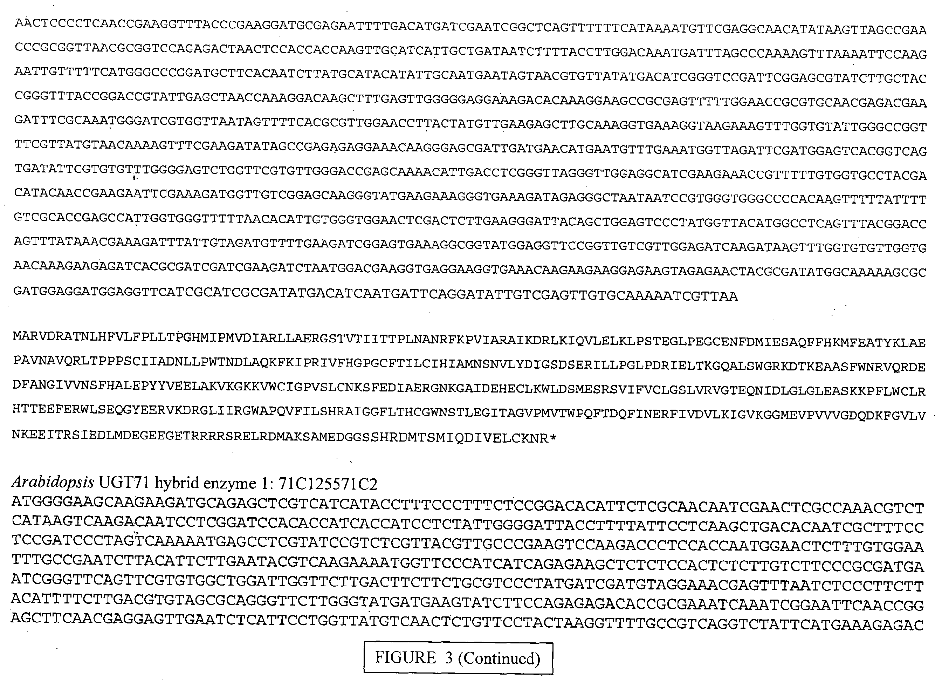

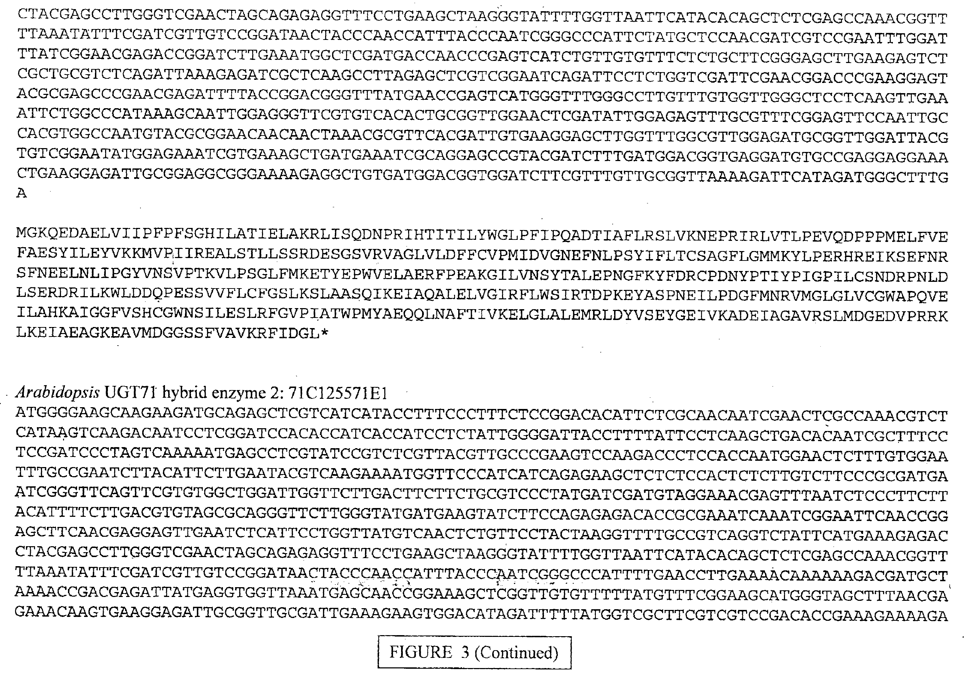

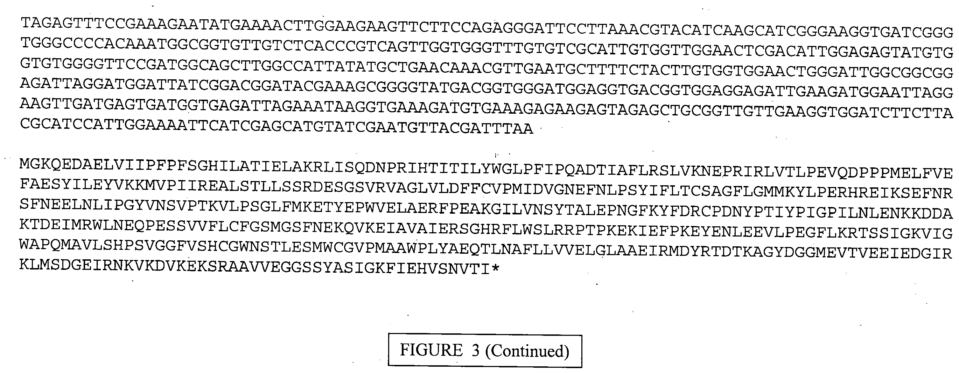

In another aspect, this document features an isolated polypeptide having at least 80% sequence identity to the UGT73 amino acid sequence set forth in FIG. 3. The polypeptide can have at least 90% sequence identity to the UGT73 amino acid sequence set forth in FIG. 3. The polypeptide can have at least 95% sequence identity to the UGT73 amino acid sequence set forth in FIG. 3. The polypeptide can have the UGT73 amino acid sequence set forth in FIG. 3.

In another aspect, this document features an isolated polypeptide having the amino acid sequence set forth in FIG. 9 and a nucleic acid encoding such a polypeptide.

This document also features a method of producing crocetin. The method includes contacting crocetin dialdehyde with an aldehyde dehydrogenase to produce crocetin.

Disclosed herein is a synthetic DNA sequence as set forth SEQ ID NO: 58 encoding the amino acid sequence as set forth in SEQ ID NO: 57.

Also disclosed herein is a synthetic DNA sequence as set forth SEQ ID NO: 65 encoding the amino acid sequence as set forth in SEQ ID NO: 66.

Unless otherwise defined, all technical and scientific terms used herein have the same meaning as commonly understood by one of ordinary skill in the art to which the invention belongs. Although methods and materials similar or equivalent to those described herein can be used to practice the invention, suitable methods and materials are described below.

In case of conflict, the present specification, including definitions, will control. In addition, the materials, methods, and examples are illustrative only and are not intended to be limiting. Other features and advantages of the invention will be apparent from the following detailed description. Applicants reserve the right to alternatively claim any disclosed invention using the transitional phrase "comprising," "consisting essentially of," or "consisting of," according to standard practice in patent law.

### DESCRIPTION OF DRAWINGS

FIG. 1 is a schematic of the biosynthetic pathway from IPP to β-carotene.
FIG. 2 is a schematic of biosynthetic pathways within saffron.
FIG. 3 contains the nucleotide and amino acid sequences of the *Stevia rebaudiana* UGT88B1 (SEQ ID NOs: 1 and 2), UGT76G1 (SEQ ID NOs: 3 and 4), UGT74G1 (SEQ ID NOs: 5 and 6), UGT91D2e (SEQ ID NOs: 7 and 8), UGT85C2 (SEQ ID NOs:.9 and 10), and UGT73 (SEQ ID NOs: 11 and 12), *Catharanthus roseus* UGT2 (SEQ ID NOs: 13 and 14), *Arabidopsis thaliana* UGT75B1 (SEQ ID NOs: 15 and 16), and two *A. thaliana* hybrid UGTs (UGT71 hybrid enzyme 1: 71C125571C2, SEQ ID NOs: 17 and 18) and UGT71 hybrid enzyme 2: 71C125571E1, SEQ ID NOs: 19 and 20).
FIG. 4 is a schematic depicting that the amino acid sequences of the UN1671, UN3356, UN4522, UN4666, UN6460, and UN2281 UGTs cluster with known UGT91 sequences.
FIG. 5 contains the sequences of the UGTs identified in Example 4 (UN6338, SEQ ID NO:21; UN4666, SEQ ID NOs: 22 (DNA) and 23 (amino acid); UN3356, SEQ ID NOs:24 (DNA) and 25 (amino acid); UN6428, SEQ ID NO:26; UN3131, SEQ ID NO:27; UN1671, SEQ ID NOs:28 (DNA) and 29 (amino acid); UN4522, SEQ ID NOs:30 (DNA) and 31 (amino acid); UN6460, SEQ ID NOs. 32 (DNA) and 33 (amino acid); UN2281, SEQ ID NOs. 34 (DNA) and 35 (amino acid); and UN2644, SEQ ID NO:36).
FIG. 6 contains the sequences of codon optimized nucleotide sequences for expression of EUGT1-EUGT19 in *Saccharomyces cerevisiae* (Source: DNA 2.0>), SEQ ID NOs. 37-55.
FIG. 7 contains the nucleotide (SEQ ID NO: 56) and amino acid sequences (SEQ ID NO: 57) of the *Crocus sativus* glucosyltransferase 2 (UGT2) (GenBank Accession No. AY262037.1), as well as codon-optimized nucleic acid sequence (SEQ ID NO: 58).
FIG. 8 contains codon optimized gene sequences used in Example 6 (SEQ ID NOs: 59-64). Lowercase sequence is extraneous to the coding region, and is used for cloning purposes.
FIG. 9 contains codon optimized nucleotide sequences (Source: GenScript) (SEQ ID NO: 65) and amino acid sequence (SEQ ID NO: 66) of the variant Crocus UGT (Cs VrUGT2) used in Example 8.
FIG. 10 contains an alignment of CsUGT2 (GenBank Accession Number: AY262037.1) and variant Cs VrUGT2 from *Crocus sativus*, as well as the amino acid sequence of each polypeptide (SEQ ID NOs. 57 and 66).
FIG. 11 contains the nucleotide sequences encoding aldehyde dehydrogenase (ALD) 2, ALD3, ALD4, ALD5, ALD6, and HFD1 (also predicted to be an aldehyde dehydrogenase) (SEQ ID NOs. 67-72).

Like reference symbols in the various drawings indicate like elements.

### DETAILED DESCRIPTION

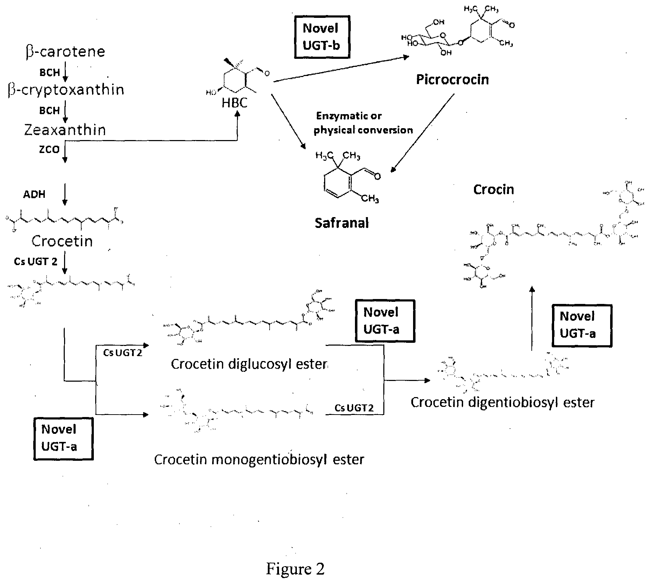

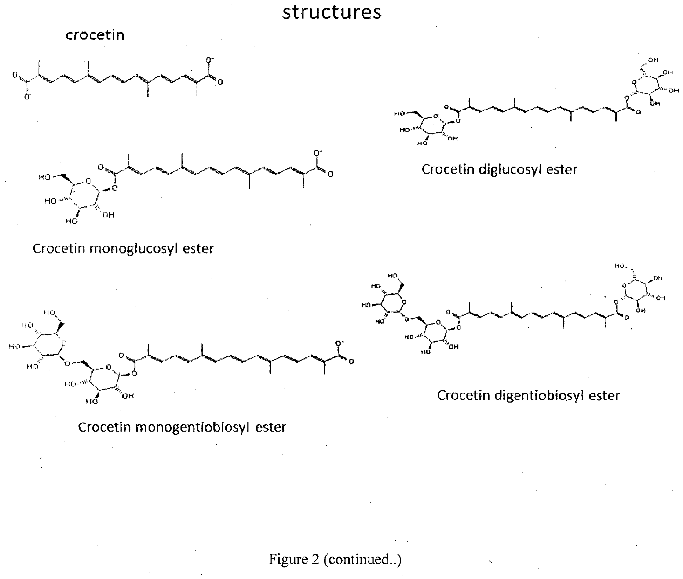

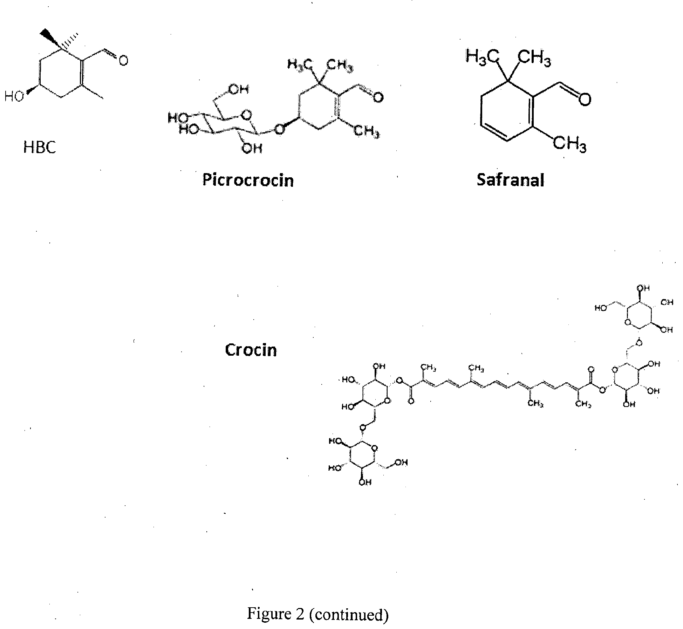

Various crocetin esters are responsible for the colorant properties of saffron extracts. Crocetin is a diterpene formed from a C18 backbone with 2 carboxylic acid groups at either end. Crocetin is derived from the carotenoid pathway containing β-carotene and zeaxanthin (see FIG. 2). The main pigment of saffron is crocin, a crocetin diester with two gentiobiose moieties (a digentiobioside). Crocin is the predominant form of the esters of crocetin. Other glycosidic forms of crocetin (also called α-crocetin or crocetin-1) include gentiobioside, glucoside, gentioglucoside, and diglucoside. γ-crocetin in the mono- or di-methylester form is also present in the saffron, along with 13-cis-crocetin, and trans crocetin isomers.

Picrocrocin, which is colorless, is responsible for the bitter taste of saffron. It is a monoterpene aldehyde produced from zeaxanthin via HBC. Deglucosylation of picrocrocin results in safranal (4-hydroxy-2,4,4-trimethyl 1-cyclohexene-1-carboxaldehyde, or dehydro-β-cyclocitral), the main aroma component of the saffron spice.

Saffron extracts also contain waxes and fats, protein, essential oils, anthocyanins, flavonoids, vitamins (riboflavin and thiamine), amino acids, starch, minerals, gums. Monoterpene aldehydes and isophorone-related compounds are volatile components of saffron, along with safranal.

This document is based on the discovery that recombinant hosts such as plant cells, plants, or microorganisms can be developed that express polypeptides useful for the biosynthesis of compounds from saffron such as crocetin, crocetin dialdehyde, picrocrocin, crocin, or safranal. Such hosts can express a zeaxanthin cleavage dioxygenase (ZCD) (also referred to as zeaxanthin cleavage oxygenase (ZCO) (e.g., from *Crocus sativus*), and in some embodiments, one or more Uridine 5'-diphospho (UDP) glycosyl transferases. Expression of these biosynthetic polypeptides in various microbial chassis allows compounds from saffron such as crocetin, crocetin dialdehyde, picrocrocin, crocin, or safranal to be produced in a consistent, reproducible manner from energy and carbon sources such as sugars, glycerol, CO₂, H₂, and sunlight. The proportion of each compound produced by a recombinant host can be tailored by incorporating preselected biosynthetic enzymes into the hosts and expressing them at appropriate levels.

At least one of the genes is a recombinant gene, the particular recombinant gene(s) depending on the species or strain selected for use. Additional genes or biosynthetic modules can be included in order to increase compound yield, improve efficiency with which energy and carbon sources are converted to saffron compounds, and/or to enhance productivity from the cell culture or plant. Such additional biosynthetic modules include genes involved in the synthesis of the terpenoid precursors, isopentenyl diphosphate and dimethylallyl diphosphate. Additional biosynthetic modules include terpene synthase and terpene cyclase genes, such as genes encoding geranylgeranyl diphosphate synthase, and genes encoding enzymes involved in caroteinoid synthesis; these genes may be endogenous genes or recombinant genes (e.g., an exogenous nucleic acid).

### Glucose to IPP

In some embodiments, a recombinant host described herein expresses recombinant genes involved in diterpene biosynthesis or production of terpenoid precursors, e.g., genes in the methylerythritol 4-phosphate (MEP) or mevalonate (MEV) pathway. For example, a recombinant host can include one or more genes encoding enzymes involved in the MEP pathway for isoprenoid biosynthesis. Enzymes in the MEP pathway include deoxyxylulose 5-phosphate synthase (DXS), D-1-deoxyxylulose 5-phosphate reductoisomerase (DXR), 4-diphosphocytidyl-2-C-methyl-D-erythritol synthase (CMS), 4-diphosphocytidyl-2-C-methyl-D-erythritol kinase (CMK), 4-diphosphocytidyl-2-C-methyl-D-erythritol 2,4-cyclodiphosphate synthase (MCS), 1-hydroxy-2-methyl-2(E)-butenyl 4-diphosphate synthase (HDS) and 1-hydroxy-2-methyl-2(E)-butenyl 4-diphosphate reductase (HDR). One or more DXS genes, DXR genes, CMS genes, CMK genes, MCS genes, HDS genes and/or HDR genes can be incorporated into a recombinant microorganism. See, Rodriguez-Concepción and Boronat, Plant Phys. 130: 1079-1089 (2002).

Suitable genes encoding DXS, DXR, CMS, CMK, MCS, HDS and/or HDR polypeptides include those made by *E*. *coli*, *Arabidopsis thaliana* and *Synechococcus leopoliensis.* Nucleotide sequences encoding DXR polypeptides are described, for example, in U.S. Patent No. 7,335,815.

In some embodiments, a recombinant host contains one or more genes encoding enzymes involved in the mevalonate pathway for isoprenoid biosynthesis. Genes suitable for transformation into a host encode enzymes in the mevalonate pathway such as a truncated 3-hydroxy-3-methyl-glutaryl (HMG)-CoA reductase (tHMG), and/or a gene encoding a mevalonate kinase (MK), and/or a gene encoding a phosphomevalonate kinase (PMK), and/or a gene encoding a mevalonate pyrophosphate decarboxylase (MPPD). Thus, one or more HMG-CoA reductase genes, MK genes, PMK genes, and/or MPPD genes can be incorporated into a recombinant host such as a microorganism.

Suitable genes encoding mevalonate pathway polypeptides are known. For example, suitable polypeptides include those made by *E*. *coli, Paracoccus denitrificans, Saccharomyces cerevisiae, Arabidopsis thaliana*, *Kitasatospora griseola*, *Homo sapiens, Drosophila melanogaster*, *Gallus gallus*, *Streptomyces sp.* KO-3988, *Nicotiana attenuata*, *Kitasatospora griseola*, *Hevea brasiliensis, Enterococcus faecium*, and *Haematococcus pluvialis.* See, e.g., U.S. Patent Nos. 7,183,089. 5,460,949, and 5,306,862.

### IPP to β-carotene

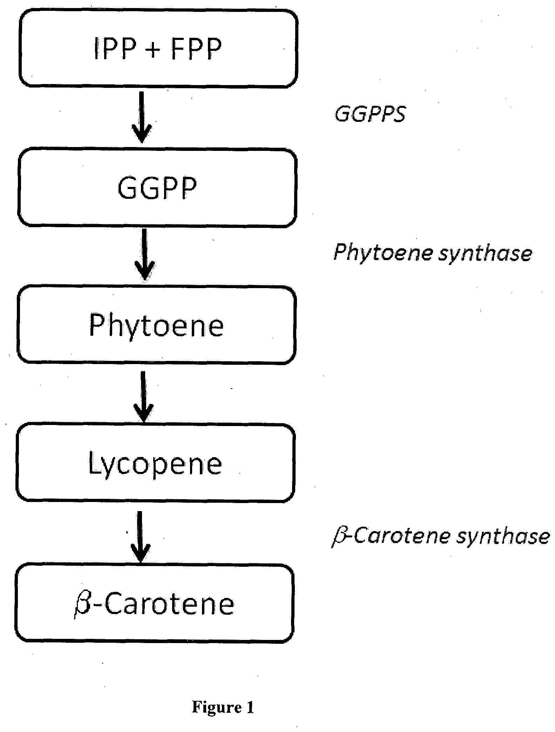

In some embodiments, a recombinant host described herein expresses genes involved in the biosynthetic pathway from IPP to β-carotene (FIG. 1). The genes may be endogenous to the host (i.e., the host naturally produces carotenoids) or can be exogenous, e.g., a recombinant gene (i.e., the host does not naturally produce carotenoids). The first step in the biosynthetic pathway from IPP to β-carotene is catalyzed by geranylgeranyl diphosphate synthase (GGPPS or also known as GGDPS, GGDP synthase, geranylgeranyl pyrophosphate synthetase or CrtE), classified as EC 2.5.1.29. In the reaction catalyzed by EC 2.5.1.29, trans,trans-farnesyl diphosphate and isopentenyl diphosphate are converted to diphosphate and geranylgeranyl diphosphate. Thus, in some embodiments, a recombinant host comprises a nucleic acid encoding GGPPS. Suitable GGPPS polypeptides are known. For example, non-limiting suitable GGPPS enzymes include those made by *Stevia rebaudiana*, *Gibberella fujikuroi*, *Mus musculus*, *Thalassiosira pseudonana*, *Xanthophyllomyces dendrorhous*, *Streptomyces clavuligerus*, *Sulfulobus acidicaldarius*, *Synechococcus sp.* and *Arabidopsis thaliana*. See, GenBank Accession Nos. ABD92926; CAA75568; AAH69913; XP_002288339; ZP_05004570; BAA43200; ABC98596; and NP_195399.

The next step in the pathway of FIG. 1 is catalyzed by phytoene synthase or CrtB, classified as EC 2.5.1.32. In this reaction catalyzed by EC 2.5.1.32, two geranylgeranyl diphosphate molecules react to form 2 pyrophosphate molecules and phytoene. This step also may be catalyzed by enzymes known as phytoene-β-carotene synthase or CrtYB. Thus, in some embodiments a recombinant host comprises a nucleic acid encoding phytoene synthase. Non-limiting examples of suitable phytoene synthases include the *X. dendrorhous* phytoene-β-carotene synthase.

The next step in the biosynthesis of β-carotene is catalyzed by phytoene dehydrogenase, also known as phytoene desaturase or Crtl. This enzyme converts phytoene to lycopene. Thus, in some embodiments a recombinant host comprises a nucleic acid encoding a phytoene dehydrogenase. Non-limiting examples of suitable phytoene dehydrogenases include *Neurospora crassa* phytoene desaturase (GenBank Accession no. XP_964713). These enzymes are also found abundantly in plants and cyanobacterium.

β-carotene is formed from lycopene with the enzyme β-carotene synthase, also called CrtY or CrtL-b. This step may also be catalyzed by the multifunctional CrtYB. Thus, in some embodiments, a recombinant host comprises a nucleic acid encoding a β-carotene synthase.

### β-carotene to zeaxanthin and saffron compounds

FIG. 2 illustrates the pathways from β-carotene to various saffron compounds.

In the initial step, β-carotene is converted to zeaxanthin. This conversion is catalyzed by β-carotene hydroxylase (BCH), which converts β-carotene to β-cryptoxanthin, which then further reacts to form zeaxanthin. This enzyme is also known as CrtZ. Suitable β-carotene hydroxylases are available from *Xanthophyllomyces dendrorhous, Arabidopsis thaliana*, *Adonis aestivalis*, as well as a number of other carotenoid producing microorganisms.

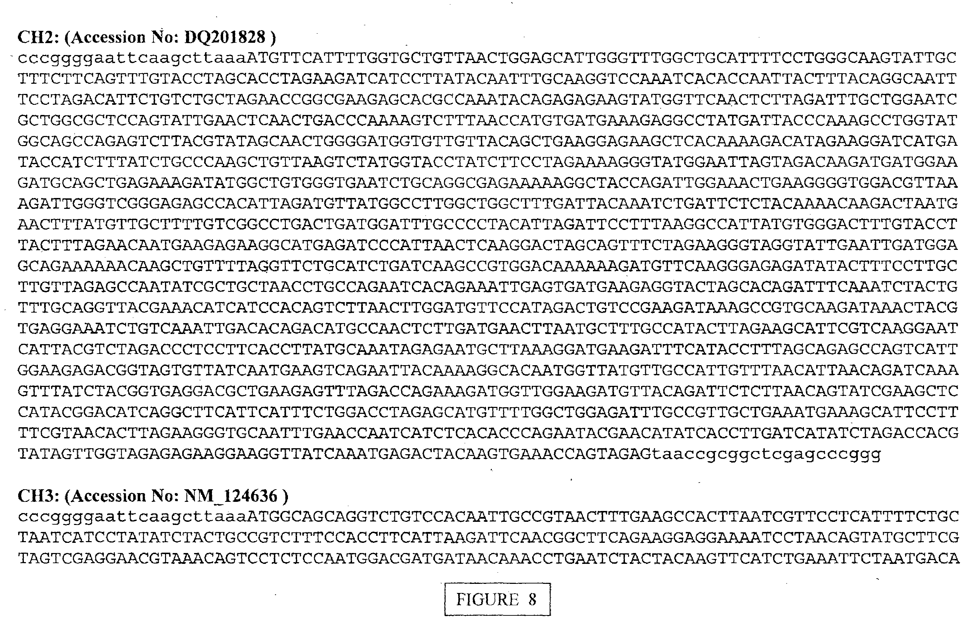

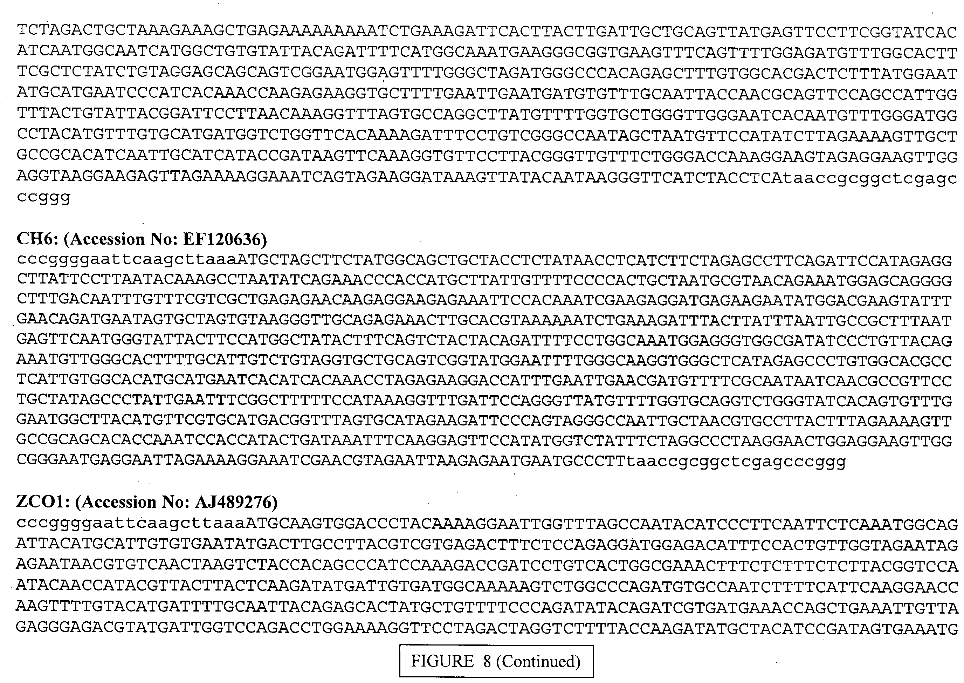

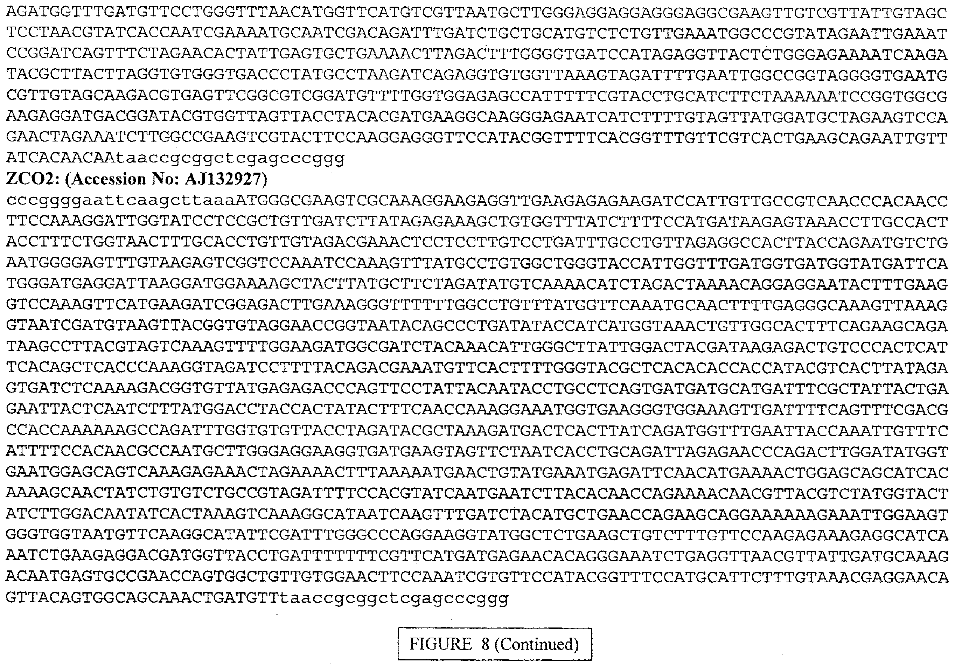

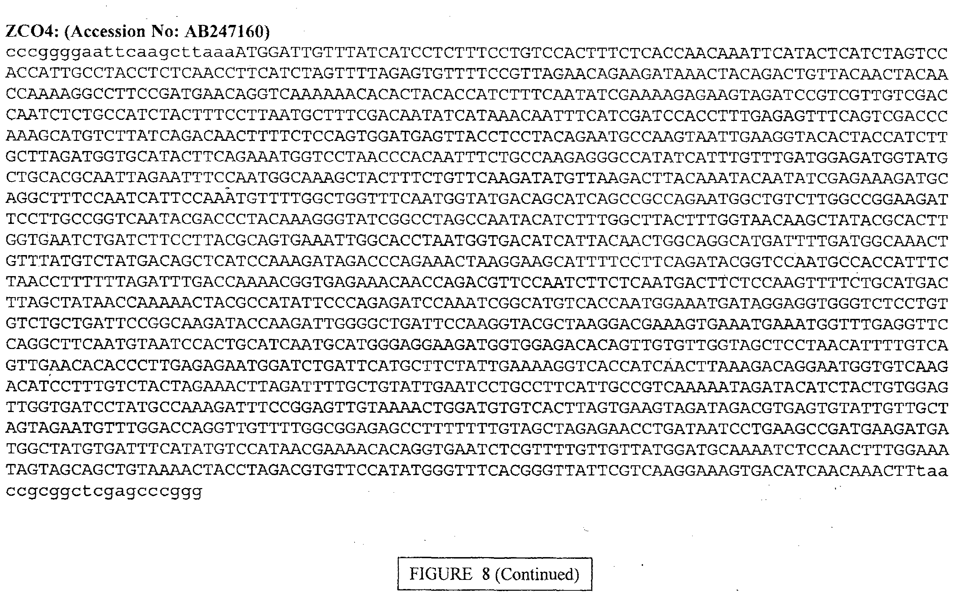

Zeaxanthin is converted to hydroxyl-β-cyclocitral (HBC) and crocetin dialdehyde via the enzyme zeaxanthin cleavage dioxygenase (ZCD) (also known as zeaxthanin cleavage oxygenase (ZCO)). A suitable ZCD is available from the *Crocus sativa* plant. See, Example 6. FIG. 8 contains a codon optimized gene sequence encoding a suitable ZCD.

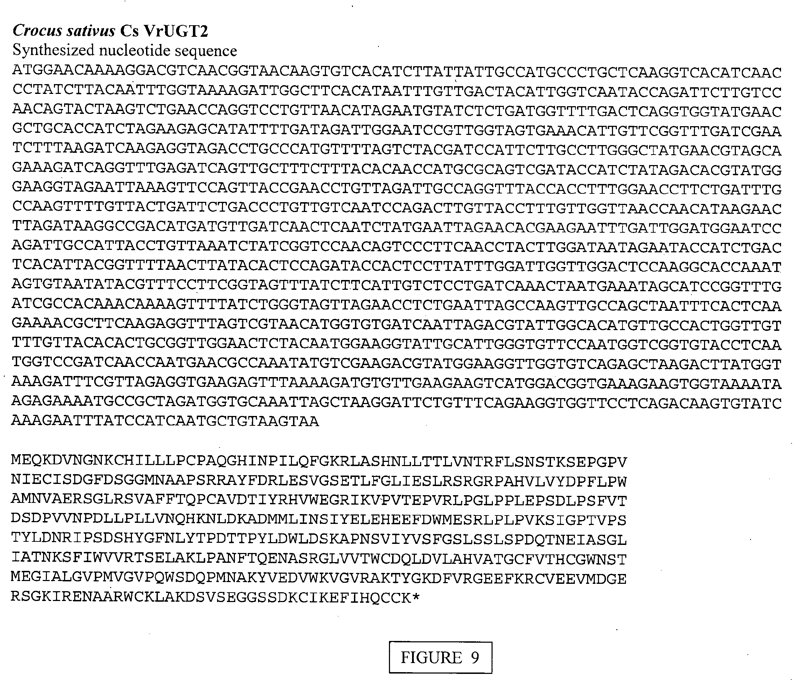

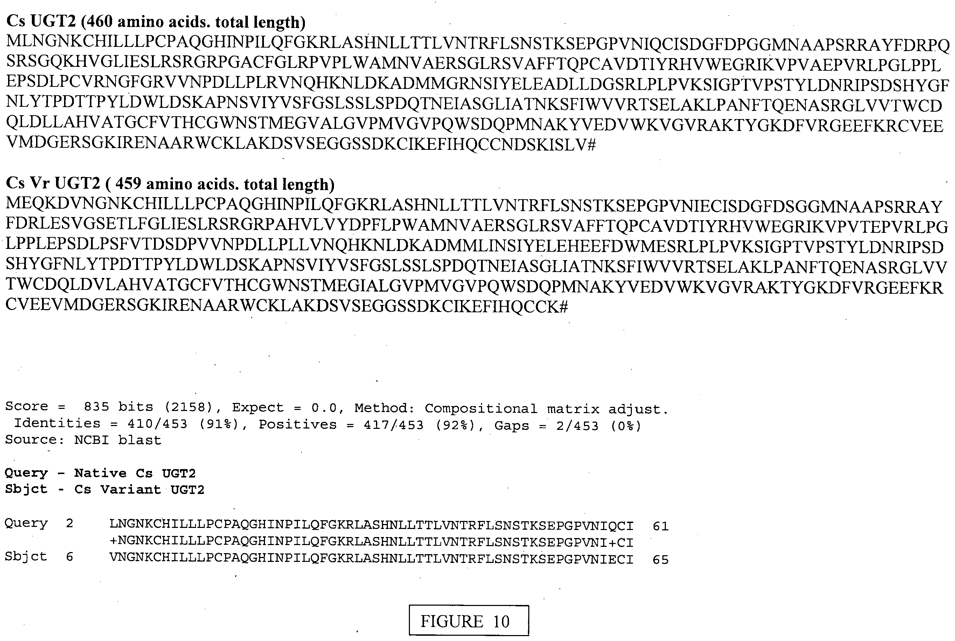

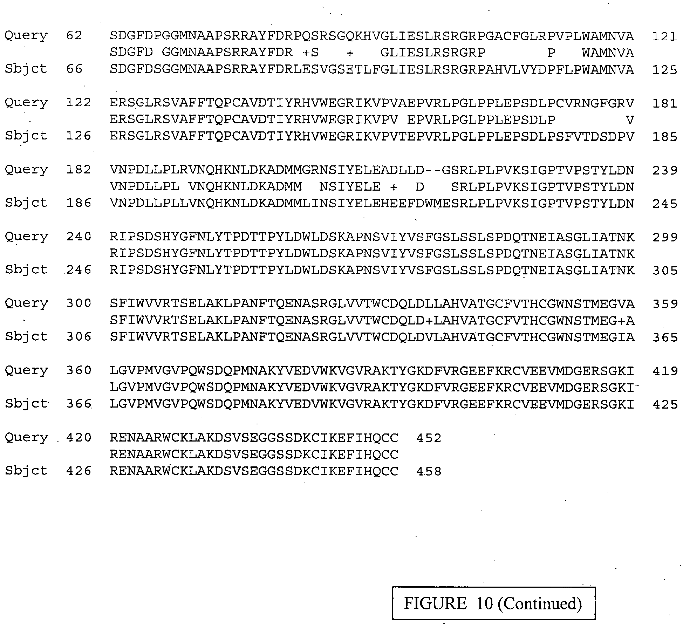

HBC is converted to picrocrocin with an aglycone O-glycosyl UGT enzyme that utilizes UDP-glucose as the glucose donor. Suitable UGTs includes UGT85C2 from *Stevia rebaudiana*, a Stevia 73-homolog, and two UGT family 71 hybrid UGTs. See, FIG. 3 for the nucleotide and amino acid sequences of these UGTs (SEQ ID NOs. 1-20). The variant Cs UGT2 also can be used (see FIGs. 9 and 10). These enzymes are referred to as UGTb in FIG. 2. The reverse reaction is catalyzed by an unknown glucosidase. To improve yields and titers for production of picrocrocin, it may be desirable to knock out β-glucosidase functionalities within the host organism of choice.

Safranal spontaneously forms during processing of saffron, it is unknown if it is due to physical conversions or requires catalysis by an enzyme or enzymes. It is unknown if HBC can be directly converted to safranal via a dehydration or if picrocrocin is an intermediate.

Crocetin dialdehyde is likely converted to crocetin in the saffron plant by an aldehyde dehydrogenase (ADH), also known as an aldehyde oxidoreductase. As described in Example 9, *S*. *cerevisiae* has multiple endogenous aldehyde dehydrogenase genes that can be used to covert the dialdehyde to the carboxylate form without introduction of heterologous genes. See Example 9.

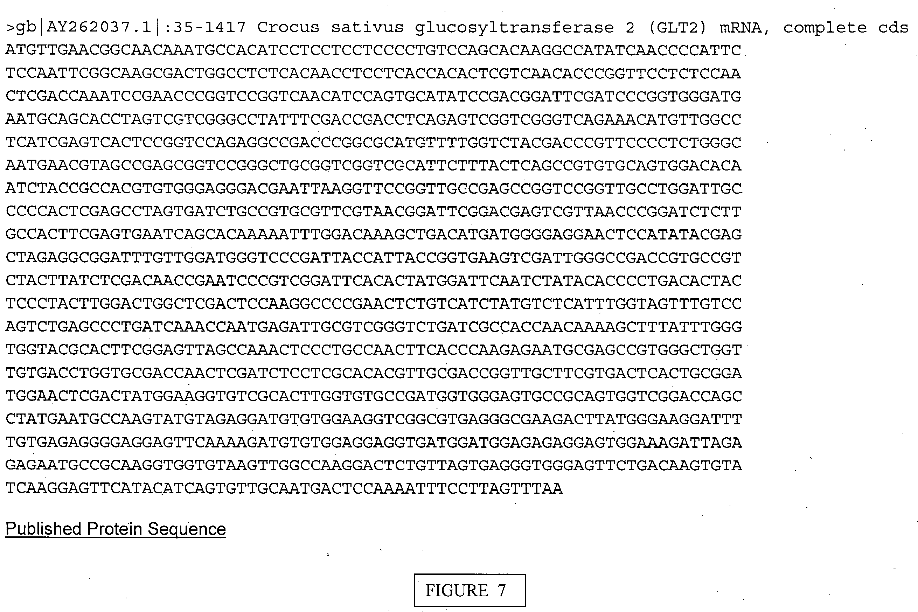

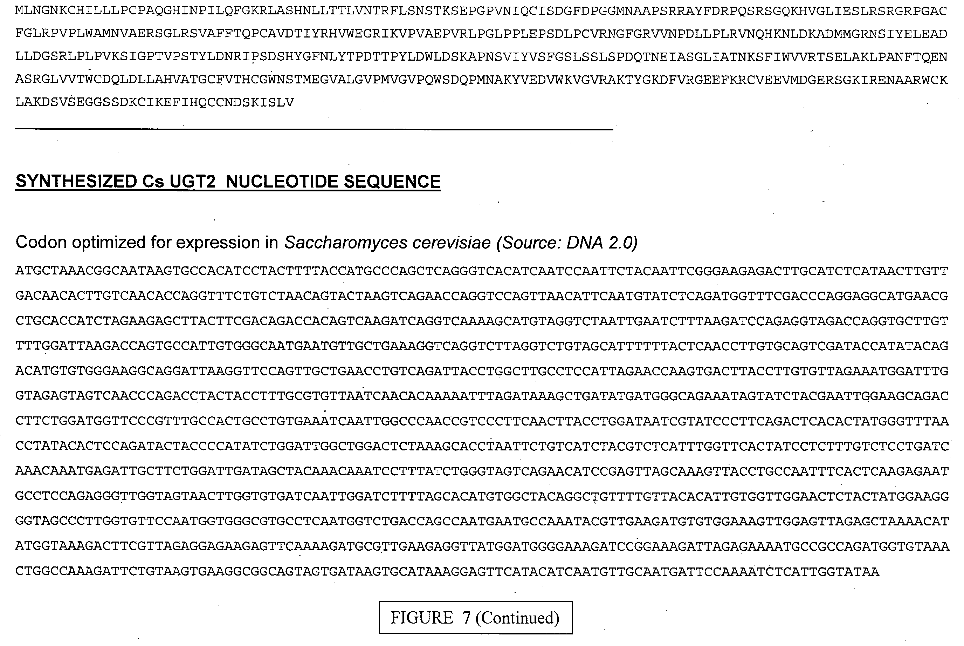

The second step in crocin formation is the addition of glucose moieties to the carboxylic acid ends of the crocetin molecule. *Crocus sativus* UGT2 (CsUGT2) has been shown to convert crocetin to monoglucosides of crocetin (crocetin monoglucosyl ester or crocetin diglyosyl ester). This enzyme is classified as EC 2.4.1, a Uridine-5'-diphosphoglucose (UDP-glucose)-crocetin 8,8'-glucosyltransferase. As such, a recombinant host can include a nucleic acid encoding a UGT2. See FIG. 7 for the nucleic acid and amino acid sequence of the *Crocus sativus* UGT2, and a codon-optimized nucleic acid sequence. The GenBank Accession Number for the CsUGT2 is AY262037.1.

A recombinant host also can include a *Crocus sativus* UGT (Cs VrUGT2) that catalyzes the formation of glucose esters (crocetin monoglucosyl ester or crocetin diglyosyl ester) from crocetin. See Example 8. The amino acid sequence of Cs VrUGT2 is provided in FIG 9. See also FIG. 10 for an alignment of Cs VrUGT2 and Cs UGT2.

A recombinant host also can include a UGT that catalyzes a β glucosyl linkage (e.g., β-1,6 glucosyl linkage) between two glucose moieties such that crocin can be formed from crocetin dialdehyde. This UGT is referred to as UGTa in FIG. 2. As such, a recombinant host can include a nucleic acid encoding a UGT2. A *Stevia rebaudiana* UGT, UGT76G1, has been shown to be able to form a crocetin ester with four glucose moieties. See Example 4. Isomeric characterization will determine if the product is crocin or a crocin analog.

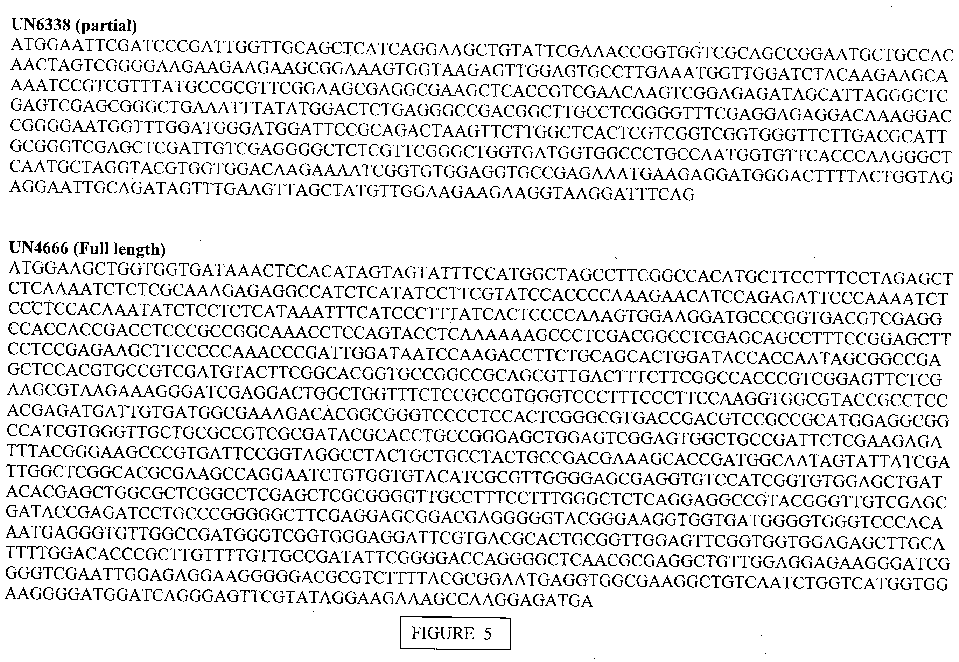

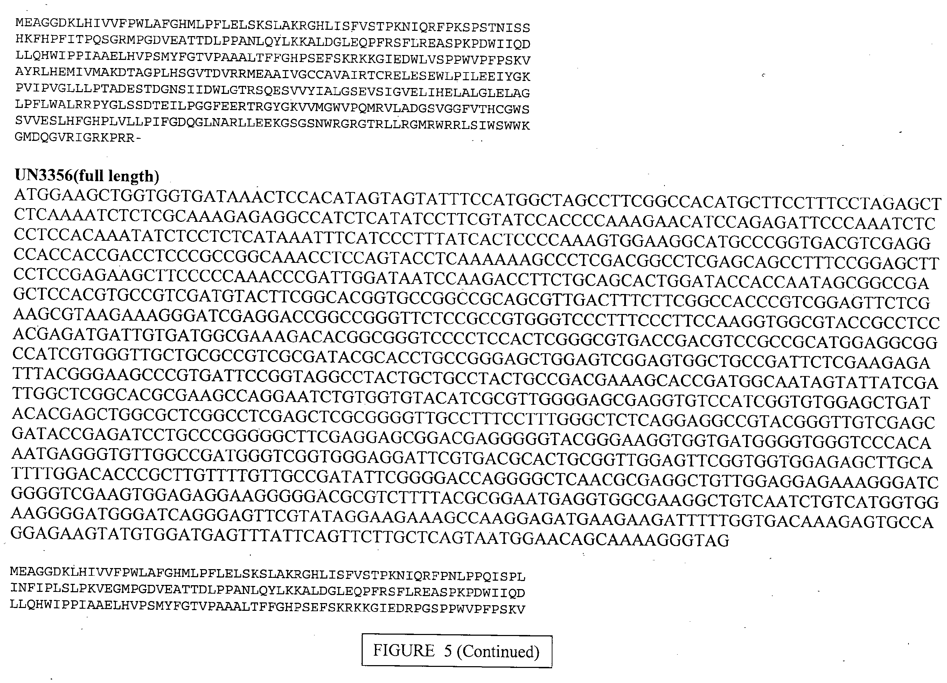

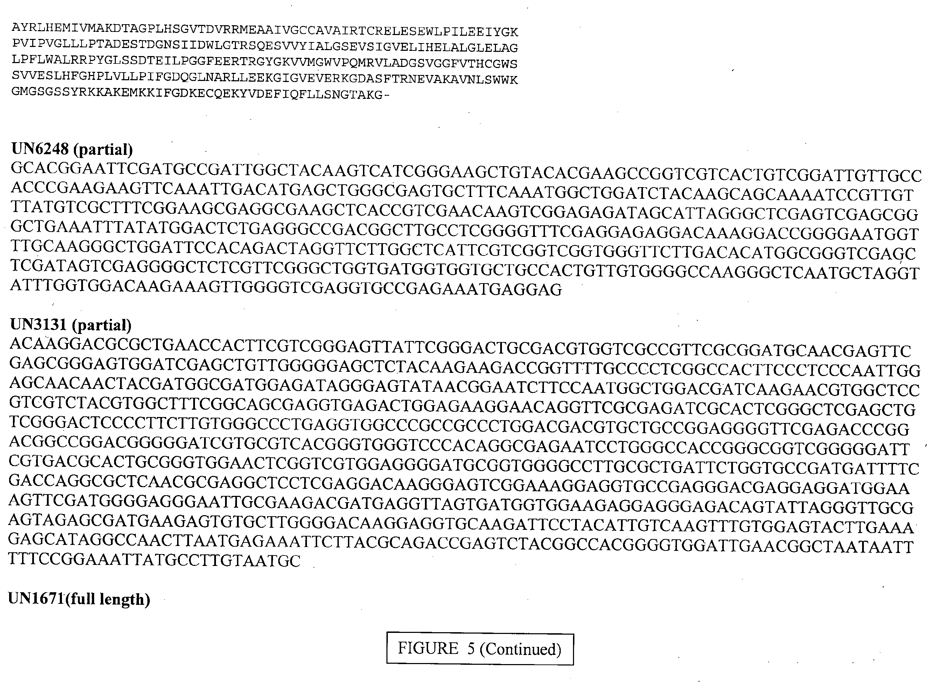

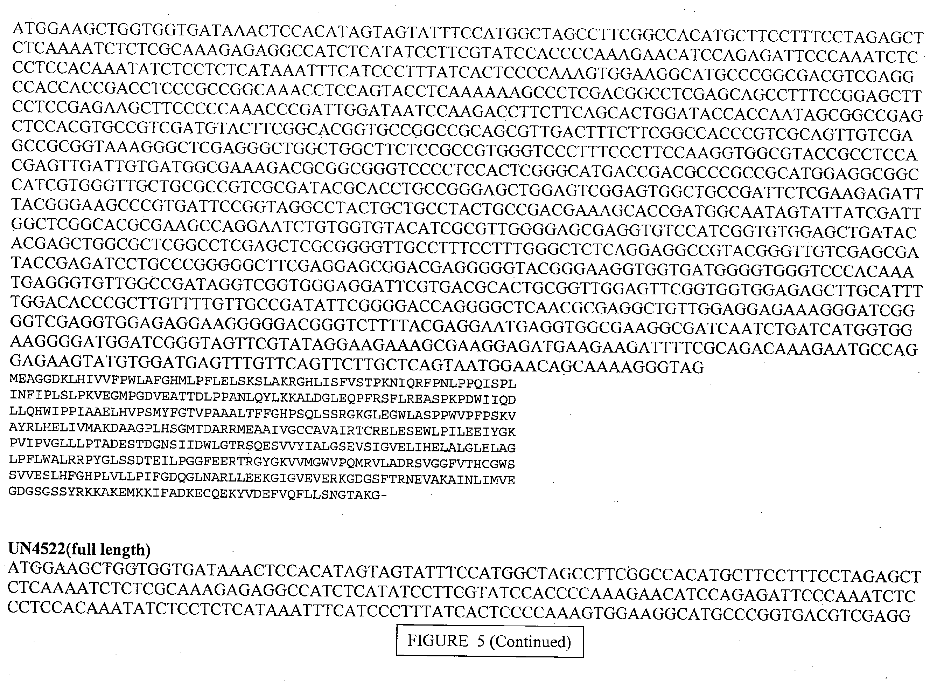

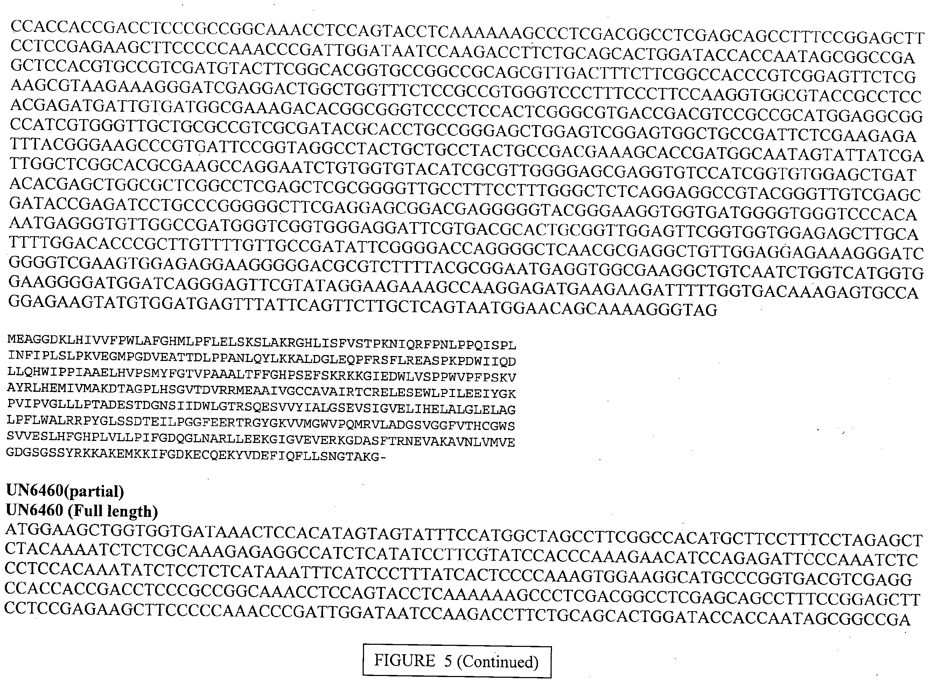

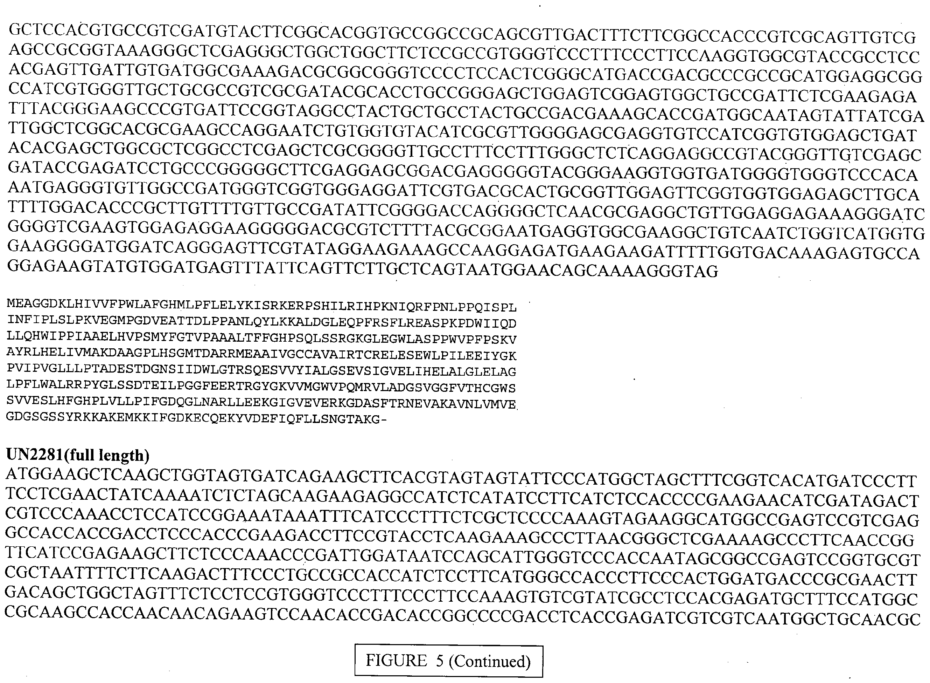

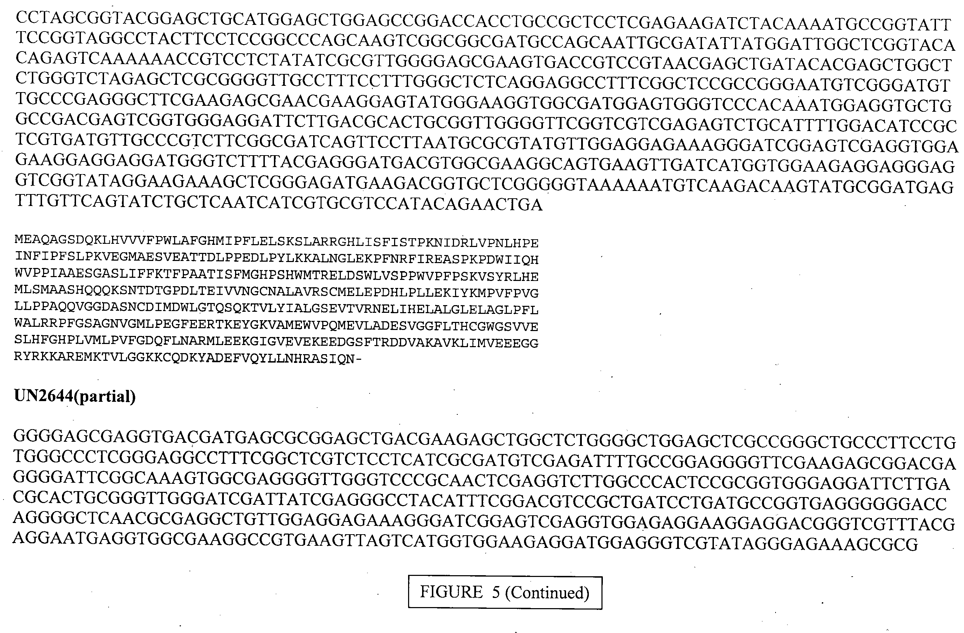

Three UGTs, UGT76G1 from *Stevia rebaudiana* and two UN1761 and UN4522 from Crocus have been shown to be able to form a crocetin ester with four glucose moieties. See Example 4. For Stevia UGT76G1, isomeric characterization will determine if the product is crocin or a crocin analog. The amino acid sequence of each of UN1761 and UN4522 is set forth in FIG 5.

A recombinant host also can include a UGT that catalyzes an aglycone crocetin at either one end or both the terminal carboxyl ends. Three UGTs UGT76G1, or UGT71 hybrid enzymes (71C125571C2 and 71C125571E1) showed the formation of mono and di glucosyl esters form crocetin. See Example 7.

A recombinant host also can include a UGT that catalyzes the formation of gentibiosyl ester directly from Crocetin. Two UGTs UGT71 hybrid enzymes (71C125571C2 and 71C125571E1) showed the formation of gentibiosyl ester from crocetin. See Example 7.

### Functional Homologs

Functional homologs of the polypeptides described above are also suitable for use in producing saffron compounds in a recombinant host. A functional homolog is a polypeptide that has sequence similarity to a reference polypeptide, and that carries out one or more of the biochemical or physiological function(s) of the reference polypeptide. A functional homolog and the reference polypeptide may be natural occurring polypeptides, and the sequence similarity may be due to convergent or divergent evolutionary events. As such, functional homologs are sometimes designated in the literature as homologs, or orthologs, or paralogs. Variants of a naturally occurring functional homolog, such as polypeptides encoded by mutants of a wild type coding sequence, may themselves be functional homologs. Functional homologs can also be created via site-directed mutagenesis of the coding sequence for a polypeptide, or by combining domains from the coding sequences for different naturally-occurring polypeptides ("domain swapping"). Techniques for modifying genes encoding functional UGT polypeptides described herein are known and include, *inter alia*, directed evolution techniques, site-directed mutagenesis techniques and random mutagenesis techniques, and can be useful to increase specific activity of a polypeptide, alter substrate specificity, alter expression levels, alter subcellular location, or modify polypeptide:polypeptide interactions in a desired manner. Such modified polypeptides are considered functional homologs. The term "functional homolog" is sometimes applied to the nucleic acid that encodes a functionally homologous polypeptide.

Functional homologs can be identified by analysis of nucleotide and polypeptide sequence alignments. For example, performing a query on a database of nucleotide or polypeptide sequences can identify homologs of polypeptides described herein. Sequence analysis can involve BLAST, Reciprocal BLAST, or PSI-BLAST analysis of nonredundant databases using the amino acid sequence of interest as the reference sequence. Amino acid sequence is, in some instances, deduced from the nucleotide sequence. Those polypeptides in the database that have greater than 40% sequence identity are candidates for further evaluation for suitability as polypeptide useful in the synthesis of compounds from saffron. Amino acid sequence similarity allows for conservative amino acid substitutions, such as substitution of one hydrophobic residue for another or substitution of one polar residue for another. If desired, manual inspection of such candidates can be carried out in order to narrow the number of candidates to be further evaluated. Manual inspection can be performed by selecting those candidates that appear to have conserved functional domains.

Conserved regions can be identified by locating a region within the primary amino acid sequence of a polypeptide described herein that is a repeated sequence, forms some secondary structure (*e.g*., helices and beta sheets), establishes positively or negatively charged domains, or represents a protein motif or domain. See, *e.g*., the Pfam web site describing consensus sequences for a variety of protein motifs and domains on the World Wide Web at sanger.ac.uk/Software/Pfam/ and pfam.janelia.org/. The information included at the Pfam database is described in Sonnhammer et al., Nucl. Acids Res., 26:320-322 (1998); Sonnhammer et al., Proteins, 28:405-420 (1997); and Bateman et al., Nucl. Acids Res., 27:260-262 (1999). Conserved regions also can be determined by aligning sequences of the same or related polypeptides from closely related species. Closely related species preferably are from the same family. In some embodiments, alignment of sequences from two different species is adequate.

Typically, polypeptides that exhibit at least about 40% amino acid sequence identity are useful to identify conserved regions. Conserved regions of related polypeptides exhibit at least 45% amino acid sequence identity (*e.g*., at least 50%, at least 60%, at least 70%, at least 80%, or at least 90% amino acid sequence identity). In some embodiments, a conserved region exhibits at least 92%, 94%, 96%, 98%, or 99% amino acid sequence identity.

A percent identity for any candidate nucleic acid or polypeptide relative to a reference nucleic acid or polypeptide can be determined as follows. A reference sequence (*e*.*g*., a nucleic acid sequence or an amino acid sequence) is aligned to one or more candidate sequences using the computer program ClustalW (version 1.83, default parameters), which allows alignments of nucleic acid or polypeptide sequences to be carried out across their entire length (global alignment). Chenna et al., Nucleic Acids Res., 31(13):3497-500 (2003).

ClustalW calculates the best match between a reference and one or more candidate sequences, and aligns them so that identities, similarities, and differences can be determined. Gaps of one or more residues can be inserted into a reference sequence, a candidate sequence, or both, to maximize sequence alignments. For fast pairwise alignment of nucleic acid sequences, the following default parameters are used: word size: 2; window size: 4; scoring method: percentage; number of top diagonals: 4; and gap penalty: 5. For multiple alignment of nucleic acid sequences, the following parameters are used: gap opening penalty: 10.0; gap extension penalty: 5.0; and weight transitions: yes. For fast pairwise alignment of protein sequences, the following parameters are used: word size: 1; window size: 5; scoring method: percentage; number of top diagonals: 5; gap penalty: 3. For multiple alignment of protein sequences, the following parameters are used: weight matrix: blosum; gap opening penalty: 10.0; gap extension penalty: 0.05; hydrophilic gaps: on; hydrophilic residues: Gly, Pro, Ser, Asn, Asp, Gln, Glu, Arg, and Lys; residue-specific gap penalties: on. The ClustalW output is a sequence alignment that reflects the relationship between sequences. ClustalW can be run, for example, at the Baylor College of Medicine Search Launcher site on the World Wide Web (searchlauncher.bcm.tmc.edu/multi-align/multi-align.html) and at the European Bioinformatics Institute site on the World Wide Web (ebi.ac.uk/clustalw).

To determine percent identity of a candidate nucleic acid or amino acid sequence to a reference sequence, the sequences are aligned using ClustalW, the number of identical matches in the alignment is divided by the length of the reference sequence, and the result is multiplied by 100. It is noted that the percent identity value can be rounded to the nearest tenth. For example, 78.11, 78.12, 78.13, and 78.14 are rounded down to 78.1, while 78.15, 78.16, 78.17, 78.18, and 78.19 are rounded up to 78.2.

It will be appreciated that polypeptides described herein can include additional amino acids that are not involved in glucosylation or other enzymatic activities carried out by the enzyme, and thus such a polypeptide can be longer than would otherwise be the case. For example, a polypeptide can include a purification tag (e.g., HIS tag or GST tag), a chloroplast transit peptide, a mitochondrial transit peptide, an amyloplast peptide, signal peptide, or a secretion tag added to the amino or carboxy terminus. In some embodiments, a polypeptide includes an amino acid sequence that functions as a reporter, *e.g*., a green fluorescent protein or yellow fluorescent protein.

### Nucleic Acids

A recombinant gene encoding a polypeptide described herein comprises the coding sequence for that polypeptide, operably linked in sense orientation to one or more regulatory regions suitable for expressing the polypeptide. Because many microorganisms are capable of expressing multiple gene products from a polycistronic mRNA, multiple polypeptides can be expressed under the control of a single regulatory region for those microorganisms, if desired. A coding sequence and a regulatory region are considered to be operably linked when the regulatory region and coding sequence are positioned so that the regulatory region is effective for regulating transcription or translation of the sequence. Typically, the translation initiation site of the translational reading frame of the coding sequence is positioned between one and about fifty nucleotides downstream of the regulatory region for a monocistronic gene.

In many cases, the coding sequence for a polypeptide described herein is identified in a species other than the recombinant host, *i.e*., is a heterologous nucleic acid. Thus, if the recombinant host is a microorganism, the coding sequence can be from other prokaryotic or eukaryotic microorganisms, from plants or from animals. In some case, however, the coding sequence is a sequence that is native to the host and is being reintroduced into that organism. A native sequence can often be distinguished from the naturally occurring sequence by the presence of non-natural sequences linked to the exogenous nucleic acid, *e.g*., non-native regulatory sequences flanking a native sequence in a recombinant nucleic acid construct. In addition, stably transformed exogenous nucleic acids typically are integrated at positions other than the position where the native sequence is found.

"Regulatory region" refers to a nucleic acid having nucleotide sequences that influence transcription or translation initiation and rate, and stability and/or mobility of a transcription or translation product. Regulatory regions include, without limitation, promoter sequences, enhancer sequences, response elements, protein recognition sites, inducible elements, protein binding sequences, 5' and 3' untranslated regions (UTRs), transcriptional start sites, termination sequences, polyadenylation sequences, introns, and combinations thereof. A regulatory region typically comprises at least a core (basal) promoter. A regulatory region also may include at least one control element, such as an enhancer sequence, an upstream element, or an upstream activation region (UAR). A regulatory region is operably linked to a coding sequence by positioning the regulatory region and the coding sequence so that the regulatory region is effective for regulating transcription or translation of the sequence. For example, to operably link a coding sequence and a promoter sequence, the translation initiation site of the translational reading frame of the coding sequence is typically positioned between one and about fifty nucleotides downstream of the promoter. A regulatory region can, however, be positioned as much as about 5,000 nucleotides upstream of the translation initiation site, or about 2,000 nucleotides upstream of the transcription start site.

The choice of regulatory regions to be included depends upon several factors, including, but not limited to, efficiency, selectability, inducibility, desired expression level, and preferential expression during certain culture stages. It is a routine matter for one of skill in the art to modulate the expression of a coding sequence by appropriately selecting and positioning regulatory regions relative to the coding sequence. It will be understood that more than one regulatory region may be present, *e.g*., introns, enhancers, upstream activation regions, transcription terminators, and inducible elements.

One or more genes can be combined in a recombinant nucleic acid construct in "modules" useful for a discrete aspect of production of a compound from saffron. Combining a plurality of genes in a module, particularly a polycistronic module, facilitates the use of the module in a variety of species. For example, a zeaxanthin cleave dioxygenase, or a UGT gene cluster, can be combined in a polycistronic module such that, after insertion of a suitable regulatory region, the module can be introduced into a wide variety of species. As another example, a UGT gene cluster can be combined such that each UGT coding sequence is operably linked to a separate regulatory region, to form a UGT module. Such a module can be used in those species for which monocistronic expression is necessary or desirable. In addition to genes useful for production of compounds from saffron, a recombinant construct typically also contains an origin of replication, and one or more selectable markers for maintenance of the construct in appropriate species.

Disclosed herein is a synthetic DNA sequence as set forth SEQ ID NO: 58 encoding the amino acid sequence as set forth in SEQ ID NO: 57.

Also disclosed herein is a synthetic DNA sequence as set forth SEQ ID NO: 65 encoding the amino acid sequence as set forth in SEQ ID NO: 66.

Disclosed is a DNA expression cassette comprising the isolated nucleic acid encoding a UGT73 polypeptide having at least 80% sequence identity to the UGT73 amino acid sequence set forth in FIG. 3 or a nucleic acid construct comprising a regulatory region operably linked to said nucleic acid.

Disclosed is a DNA expression cassette comprising the synthetic DNA sequence as set forth SEQ ID NO: 58 encoding the amino acid sequence as set forth in SEQ ID NO: 57, wherein the isolated nucleic acid or synthetic DNA sequence id operably linked to a promoter.

Also disclosed is a DNA expression cassette comprising the synthetic DNA sequence as set forth SEQ ID NO: 65 encoding the amino acid sequence as set forth in SEQ ID NO: 66, wherein the isolated nucleic acid or synthetic DNA sequence id operably linked to a promoter.

Further disclosed is a recombinant vector comprising the DNA expression cassette comprising the isolated nucleic acid encoding a UGT73 polypeptide having at least 80% sequence identity to the UGT73 amino acid sequence set forth in FIG. 3 or a nucleic acid construct comprising a regulatory region operably linked to said nucleic acid.

In addition, disclosed herein is a recombinant vector comprising the DNA expression cassette a DNA expression cassette comprising the the synthetic DNA sequence as set forth SEQ ID NO: 58 encoding the amino acid sequence as set forth in SEQ ID NO: 57, wherein the isolated nucleic acid or synthetic DNA sequence id operably linked to a promoter.

Also disclosed is a recombinant vector comprising the DNA expression cassette a DNA expression cassette comprising the the synthetic DNA sequence as set forth SEQ ID NO: 65 encoding the amino acid sequence as set forth in SEQ ID NO: 66, wherein the isolated nucleic acid or synthetic DNA sequence id operably linked to a promoter.

Further disclosed is a recombinant cell comprising the DNA expression cassette or the recombinant vector as disclosed herein.

Some embodiments of the present invention relate to a recombinant cell selected from a group consisting of yeast, *E. coli,* plant cell, mammalian cell and insect cell.

Yet another embodiment of the present invention relates to a recombinant cell as wherein the recombinant cell is *Saccharomyces cerevisivae*.

It will be appreciated that because of the degeneracy of the genetic code, a number of nucleic acids can encode a particular polypeptide; *i.e*., for many amino acids, there is more than one nucleotide triplet that serves as the codon for the amino acid. Thus, codons in the coding sequence for a given polypeptide can be modified such that optimal expression in a particular host is obtained, using appropriate codon bias tables for that host (e.g., microorganism). As isolated nucleic acids, these modified sequences can exist as purified molecules and can be incorporated into a vector or a virus for use in constructing modules for recombinant nucleic acid constructs.

### Recombinant Hosts

A number of prokaryotes and eukaryotes are suitable for use in constructing the recombinant microorganisms described herein, e.g., gram-negative bacteria, yeast and fungi. A species and strain selected for use as a strain for production of saffron compounds is first analyzed to determine which production genes are endogenous to the strain and which genes are not present (e.g., carotenoid genes). Genes for which an endogenous counterpart is not present in the strain are assembled in one or more recombinant constructs, which are then transformed into the strain in order to supply the missing function(s).

Exemplary prokaryotic and eukaryotic species are described in more detail below. However, it will be appreciated that other species may be suitable. For example, suitable species may be in a genus selected from the group consisting of *Agaricus, Aspergillus, Bacillus*, *Candida*, *Corynebacterium*, *Escherichia*, *Fusarium*/*Gibberella*, *Kluyveromyces, Laetiporus*, *Lentinus, Phaffia*, *Phanerochaete*, *Pichia*, *Physcomitrella*, *Rhodoturula*, *Saccharomyces*, *Schizosaccharomyces*, *Sphaceloma*, *Xanthophyllomyces* and *Yarrowia.* Exemplary species from such genera include *Lentinus tigrinus, Laetiporus sulphureus, Phanerochaete chrysosporium*, *Pichia pastoris, Physcomitrella patens*, *Rhodoturula glutinis* 32, *Rhodoturula mucilaginosa*, *Phaffia rhodozyma* UBV-AX, *Xanthophyllomyces dendrorhous, Fusarium fujikuroi*/*Gibberella fujikuroi*, *Candida utilis* and *Yarrowia lipolytica.* In some embodiments, a microorganism can be an Ascomycete such as *Gibberella fujikuroi*, *Kluyveromyces lactis*, *Schizosaccharomyces pombe, Aspergillus niger,* or *Saccharomyces cerevisiae.* In some embodiments, a microorganism can be a prokaryote such as *Escherichia coli, Rhodobacter sphaeroides,* or *Rhodobacter capsulatus.* It will be appreciated that certain microorganisms can be used to screen and test genes of interest in a high throughput manner, while other microorganisms with desired productivity or growth characteristics can be used for large-scale production of compounds from saffron.

### Saccharomyces cerevisiae

*Saccharomyces cerevisiae* is a widely used chassis organism in synthetic biology, and can be used as the recombinant microorganism platform. There are libraries of mutants, plasmids, detailed computer models of metabolism and other information available for *S. cerevisiae*, allowing for rational design of various modules to enhance product yield. Methods are known for making recombinant microorganisms.

The genes described herein can be expressed in yeast using any of a number of known promoters. Strains that overproduce terpenes are known and can be used to increase the amount of geranylgeranyl diphosphate available for production of saffron compounds.

Suitable strains of *S*. *cerevisiae* also can be modified to allow for increased accumulation of storage lipids and/or increased amounts of available precursor molecules such as acetyl-CoA. For example, accumulation of triacylglycerols (TAG) up to 30% in *S*. *cerevisiae* was demonstrated by Kamisaka et al. (Biochem. J. (2007) 408, 61-68) by disruption of a transcriptional factor SNF2, overexpression of a plant-derived diacyl glycerol acyltransferase 1 (DGA1), and over-expression of yeast LEU2. Furthermore, Froissard et al. (FEMS Yeast Res 9 (2009) 428-438) showed that expression in yeast of AtClol, a plant oil body-forming protein, will promote oil body formation and result in over-accumulation of storage lipids. Such accumulated TAGs or fatty acids can be diverted towards acetyl-CoA biosynthesis by, for exmapple, further expressing an enzyme known to be able to form acetyl-CoA from TAG (POX genes) (e.g., a Yarrowia lipolytica POX gene).

### Aspergillus spp.

*Aspergillus* species such as *A. oryzae, A. niger* and *A. sojae* are widely used microorganisms in food production, and can also be used as the recombinant microorganism platform. Nucleotide sequences are available for genomes of *A. nidulans*, *A. fumigatus*, *A. oryzae, A. clavatus*, *A. flavus*, *A. niger,* and *A. terreus,* allowing rational design and modification of endogenous pathways to enhance flux and increase product yield. Metabolic models have been developed for *Aspergillus,* as well as transcriptomic studies and proteomics studies. *A. niger* is cultured for the industrial production of a number of food ingredients such as citric acid and gluconic acid, and thus species such as *A. niger* are generally suitable for the production of compounds from saffron.

### Escherichia coli

*Escherichia coli,* another widely used platform organism in synthetic biology, can also be used as the recombinant microorganism platform. Similar to *Saccharomyces,* there are libraries of mutants, plasmids, detailed computer models of metabolism and other information available for *E*. *coli,* allowing for rational design of various modules to enhance product yield. Methods similar to those described above for *Saccharomyces* can be used to make recombinant *E. coli* microorganisms.

### Agaricus, Gibberella, and Phanerochaete spp.

*Agaricus*, *Gibberella*, and *Phanerochaete* spp. can be useful because they are known to produce large amounts of gibberellin in culture. Thus, the terpene precursors for producing large amounts of componds from saffron are already produced by endogenous genes. Thus, modules containing recombinant genes for biosynthesis of compounds from saffron can be introduced into species from such genera without the necessity of introducing mevalonate or MEP pathway genes.

### Rhodobacter spp.

*Rhodobacter* can be use as the recombinant microorganism platform. Similar to *E*. *coli*, there are libraries of mutants available as well as suitable plasmid vectors, allowing for rational design of various modules to enhance product yield. Isoprenoid pathways have been engineered in membraneous bacterial species of *Rhodobacter* for increased production of carotenoid and CoQ10. See, U.S. Patent Publication Nos. 20050003474 and 20040078846. Methods similar to those described above for *E. coli* can be used to make recombinant *Rhodobacter* microorganisms.

### Physcomitrella spp.

*Physcomitrella* mosses, when grown in suspension culture, have characteristics similar to yeast or other fungal cultures. This genera is becoming an important type of cell for production of plant secondary metabolites, which can be difficult to produce in other types of cells.

### Plants and Plant Cells

In some embodiments, the nucleic acids and polypeptides described herein are introduced into plants or plant cells to produce compounds from saffron. Thus, a host can be a plant or a plant cell that includes at least one recombinant gene described herein. A plant or plant cell can be transformed by having a recombinant gene integrated into its genome, *i.e*., can be stably transformed. Stably transformed cells typically retain the introduced nucleic acid with each cell division. A plant or plant cell can also be transiently transformed such that the recombinant gene is not integrated into its genome. Transiently transformed cells typically lose all or some portion of the introduced nucleic acid with each cell division such that the introduced nucleic acid cannot be detected in daughter cells after a sufficient number of cell divisions. Both transiently transformed and stably transformed transgenic plants and plant cells can be useful in the methods described herein.

Transgenic plant cells used in methods described herein can constitute part or all of a whole plant. Such plants can be grown in a manner suitable for the species under consideration, either in a growth chamber, a greenhouse, or in a field. Transgenic plants can be bred as desired for a particular purpose, *e.g*., to introduce a recombinant nucleic acid into other lines, to transfer a recombinant nucleic acid to other species, or for further selection of other desirable traits. Alternatively, transgenic plants can be propagated vegetatively for those species amenable to such techniques. As used herein, a transgenic plant also refers to progeny of an initial transgenic plant provided the progeny inherits the transgene. Seeds produced by a transgenic plant can be grown and then selfed (or outcrossed and selfed) to obtain seeds homozygous for the nucleic acid construct.

Transgenic plants can be grown in suspension culture, or tissue or organ culture. For the purposes of this invention, solid and/or liquid tissue culture techniques can be used. When using solid medium, transgenic plant cells can be placed directly onto the medium or can be placed onto a filter that is then placed in contact with the medium. When using liquid medium, transgenic plant cells can be placed onto a flotation device, *e.g*., a porous membrane that contacts the liquid medium.

When transiently transformed plant cells are used, a reporter sequence encoding a reporter polypeptide having a reporter activity can be included in the transformation procedure and an assay for reporter activity or expression can be performed at a suitable time after transformation. A suitable time for conducting the assay typically is about 1-21 days after transformation, *e*.*g*., about 1-14 days, about 1-7 days, or about 1-3 days. The use of transient assays is particularly convenient for rapid analysis in different species, or to confirm expression of a heterologous polypeptide whose expression has not previously been confirmed in particular recipient cells.

Techniques for introducing nucleic acids into monocotyledonous and dicotyledonous plants are known in the art, and include, without limitation, *Agrobacterium*-mediated transformation, viral vector-mediated transformation, electroporation and particle gun transformation, U.S. Patent Nos 5,538,880; 5,204,253; 6,329,571; and 6,013,863. If a cell or cultured tissue is used as the recipient tissue for transformation, plants can be regenerated from transformed cultures if desired, by techniques known to those skilled in the art.

A population of transgenic plants can be screened and/or selected for those members of the population that have a trait or phenotype conferred by expression of the transgene. For example, a population of progeny of a single transformation event can be screened for those plants having a desired level of expression of a ZCD or UGT polypeptide or nucleic acid. Physical and biochemical methods can be used to identify expression levels. These include Southern analysis or PCR amplification for detection of a polynucleotide; Northern blots, S1 RNase protection, primer-extension, or RT-PCR amplification for detecting RNA transcripts; enzymatic assays for detecting enzyme or ribozyme activity of polypeptides and polynucleotides; and protein gel electrophoresis, Western blots, immunoprecipitation, and enzyme-linked immunoassays to detect polypeptides. Other techniques such as *in situ* hybridization, enzyme staining, and immunostaining also can be used to detect the presence or expression of polypeptides and/or nucleic acids. Methods for performing all of the referenced techniques are known. As an alternative, a population of plants comprising independent transformation events can be screened for those plants having a desired trait, such as production of a compound from saffron. Selection and/or screening can be carried out over one or more generations, and/or in more than one geographic location. In some cases, transgenic plants can be grown and selected under conditions which induce a desired phenotype or are otherwise necessary to produce a desired phenotype in a transgenic plant. In addition, selection and/or screening can be applied during a particular developmental stage in which the phenotype is expected to be exhibited by the plant. Selection and/or screening can be carried out to choose those transgenic plants having a statistically significant difference in a level of a saffron compound relative to a control plant that lacks the transgene.

The nucleic acids, recombinant genes, and constructs described herein can be used to transform a number of monocotyledonous and dicotyledonous plants and plant cell systems. Non-limiting examples of suitable monocots include, for example, cereal crops such as rice, rye, sorghum, millet, wheat, maize, and barley. The plant also may be a dicot such as soybean, cotton, sunflower, pea, geranium, spinach, or tobacco. In some cases, the plant may contain the precursor pathways for phenyl phosphate production such as the mevalonate pathway, typically found in the cytoplasm and mitochondria. The non-mevalonate pathway is more often found in plant plastids [Dubey, et al., 2003 J. Biosci. 28 637-646]. One with skill in the art may target expression of biosynthesis polypeptides to the appropriate organelle through the use of leader sequences, such that biosynthesis occurs in the desired location of the plant cell. One with skill in the art will use appropriate promoters to direct synthesis, e.g., to the leaf of a plant, if so desired. Expression may also occur in tissue cultures such as callus culture or hairy root culture, if so desired.

The invention will be further described in the following examples, which do not limit the scope of the invention described in the claims.

### EXAMPLES

Any subject matter within the examples that falls outside of the scope of the claims is provided for information purposes only.

### Example 1: Production of β-carotene in yeast

A β-carotene producing yeast reporter strain was constructed for eYAC experiments designed to find optimal combinations of saffron biosynthetic genes. The *Neurospora crassa* phytoene desaturase (also known as phytoene dehydrogenase) (accession no. XP_964713) and the *Xanthophyllomyces dendrorhous* GGDP synthase, also known as geranylgeranyl pyrophosphate synthetase or CrtE (accession no. DQ012943) and *X. dendrorhous* phytoene- β-carotene synthase CrtYB (accession no. AY177204) genes were all inserted into expression cassettes, and these expression cassettes were integrated into the genome of the laboratory yeast strain *Saccharomyces cerevisiae* CEN.PK 113-11. The phytoene desaturase and CrtYB were over-expressed under control of the strong constitutive GPD1 promoter, while overexpression of CrtE was enabled using the strong constitutive TPI1 promoter. Chromosomal integration of the *X. dendrorhous* CrtE and *Neurospora crassa* phytoene desaturase expression cassettes was done in the *S. cerevisiae* ECM3-YOR093C intergenic region while integration of the CrtYB expression cassette was done in the *S. cerevisiae* KIN1-INO2 intergenic region.

Colonies grown on SC dropout plates exhibit an orange color formation when β-carotene is produced. The presence of β-carotene is quantified by extraction into methanol and LC/MS analysis.

### Example 2: Optimized yeast production of HBC and crocetin dialdehyde

It is known that crocetin is formed from crocetin dialdehyde, and crocetin dialdehyde and hydroxyl-beta-cyclocitral (HBC) are generated upon zeaxanthin cleavage with the enzyme zeaxanthin cleavage dioxygenase (ZCD). A collection of genes were assembled in eYACs to establish an optimal pathway for biosynthesis of crocetin dialdehyde and HBC, using eYACs and the β-carotene producing yeast strain described in Example 1.

A collection of gene analogs for the enzymes that convert β -carotene into crocetin dialdehyde were sourced by yeast codon optimized synthesis (DNA 2.0), and inserted in eYAC Entry Vectors under a variety of methionine repressible gene promoters. The use of eYAC technology has been described by Naesby et al., Microb Cell Fact. 8:45 (2009). Expression cassettes for the 37 saffron biosynthesis genes shown in Table 1 were concatenated (with or without UGT genes) and ligated into eYACs. Both types of eYACs were transformed into the β-carotenoid producing yeast strain EFSC301. This strain is a stable carotenoid producer made by integration of the GPD/TPI promoter-based CrtYB/CrtE/Nc-AI-1 gene expression cassettes in the yeast ECM3 and KIN1 3'UTR regions.

A yeast transformation efficiency of approximately 800 colonies/plate was obtained using single auxotrophic selection plates. The transformants were then re-streaked on double auxotrophic selection plates (leucine-, tryptophan-). The positive transformants are grown in SC dropout media (-leucine, -tryptophan, and -methionine). Cells are grown for 24 - 72 hours at 30°C in shake flasks, and cell-free broth as well as cell extracts are extracted into organic solvent and analyzed for the presence of HBC, crocetin dialdehyde, and crocetin.

Based on the content of crocetin dialdehyde, crocetin and HBC biosynthesized in transformed yeasts, high, medium and low producers are identified. These tranformants are screened by PCR to determine gene composition of the high, medium, and low producers. Based on PCR results, the genes which are essential and non-essential for crocetin dialdehyde, crocetin and HBC production are identified and constructs can be further improved by adding or deleting genes in new combinations and in new eYAC constructs

**Table 1: Gene sources for eYAC construction**

| **S.No.** | **Code** | **Accesion No** | **Gene Name** | **Source** | **Size (bps)** |
|---|---|---|---|---|---|
| 1 | CH-1 | D90087 | β-carotene hydroxylase | *Pantoea ananatis (bacteria)* | 567 |
| 2 | CH-2 | DQ201828 | β-carotene 3-hydroxylase (crtS) | *Xanthophyllomyces dendrorhous* | 1713 |
| 3 | CH-3 | NM_124636 | β-ring hydroxylase (CHY2) | *Arabidopsis thaliana (plant)* | 951 |
| 4 | CH-4 | AF125576 | β-carotene hydroxylase | *Arabidopsis thaliana* | 972 |
| 5 | ZCO-1 | AJ489276 | zeaxanthin cleavage dioxygenase (CsZCO) | *Crocus sativus* | 1149 |
| 6 | ZCO-2 | AJ132927 | carotenoid 9,10(9',10')-cleavage dioxygenase (CsCCD) | *Crocus sativus* | 1680 |
| 7 | ZCO-3 | AJ489277 | lycopene cleavage oxygenase | *Bixa orellana (plant)* | 1149 |
| 8 | ZCO-4 | AB247160 | Carotenoid Cleavage Dioxygenase (CmCCD4a) | *[Chrysanthemum x morifolium]* | 1824 |
| 9 | ZCO-5 | AB120111 | carotenoid cleavage dioxygenase 1 (Ls CCD1) | *Lactuca sativa* | 1818 |
| 10 | ZCO-6 | EU334434 | carotenoid cleavage dioxygenase 4 | *Osmanthus fragrans* | 1869 |
| 11 | ZCO-7 | AY662342 | 9-cis-epoxy-carotenoid dioxygenase 1 | *Solanum tuberosum* | 1851 |
| 12 | EUGT-1 | AY262037 | glucosyltransferase 2, UGTCs2 | *Crocus sativus* | 1422 |
| 13 | EUGT-2 | AP003270 | putative UDP-glucosyltransferase | *Oryza sativa* | 1461 |
| 14 | EUGT-3 | AP005171 | putative UDP-glucosyltransferase | *Oryza sativa* | 1563 |
| 15 | EUGT-4 | AP005643 | putative UDP-glucosyltransferase | *Oryza sativa* | 1335 |
| 16 | EUGT-5 | AY290820 | glucosyltransferase, UGTCs3 | *Crocus sativus* | 1467 |
| 17 | CH-5 | U58919 | beta-carotene hydroxylase | *Arabidopsis thaliana* | 887 |
| 18 | CH-6 | EF120636 | β-carotene hydroxylase | *Adonis aestivalis* | 930 |
| 19 | CH-7 | Y14810 | beta-carotene hydroxylase | *Solanum lycoporsicum* | 945 |
| 20 | CH-8 | NM_001036638 | carotene beta-ring hydroxylase (BETA-OHASE 1) | *Arabidopsis thaliana* | 675 |
| 21 | CH-9 | NC_010475 | beta-carotene oxygenase CrtR | *Synechococcus sp. PCC 7002* | 888 |
| 22 | CH-10 | NC_008819 | beta-carotene hydroxylase | *Prochlorococcus marinus* | 1032 |
| 23 | CH-11 | NC_010296 | beta-carotene hydroxylase (crtR) | *Microcystis aeruginosa* | 894 |
| 24 | EUGT-6 | AP005259 | putative UDP-glucosyltransferase | *Oryza sativa* | 1539 |
| 25 | EUGT-7 | AP005171 | putative UDP-glucosyltransferase | *Oryza sativa* | 1524 |
| 26 | EUGT-8 | XM_470006 | putative UDP-glucoronosyl and UDP-glucosyl | *Oryza sativa* | 1452 |
| 27 | EUGT-9 | AP005643 | putative UDP-glucosyltransferase | *Oryza sativa* | 1296 |
| 28 | EUGT-10 | AC133334 | putative UDP-glucoronosyl and UDP-glucosyl transferase | *Oryza sativa* | 1419 |
| 29 | EUGT-11 | AC133334 | putative UDP-glucoronosyl and UDP-glucosyl transferase | *Oryza sativa* | 2319 |
| 30 | EUGT-12 | AP004741 | putative UDP-glucosyltransferase | *Oryza sative* | 1338 |
| 31 | EUGT-13 | AB012241 | glucosyltransferase-like protein | *Arabidopsis thaliana* | 1056 |
| 32 | EUGT-14 | AL133314 | glucosyltransferase-like protein | *Arabidopsis thaliana* | 1317 |
| 33 | EUGT-15 | Z25802 | UDP rhamnose: anthocyanidin-3-glucoside rhamnosyltransferase | *Petunia x hybrida* | 1416 |
| 34 | EUGT-16 | AC004786 | putative flavonol 3-0-glucosyltransfetase | *Arabidopsis thaliana* | 1329 |
| 35 | EUGT-17 | AB294391 | glucosyltransferase | *Dianthus caryophyllus* | 1386 |
| 36 | EUGT-18 | AB192314 | glucosyltransferase | *Ipomoea nil* | 1380 |
| 37 | EUGT-19 | NM_001074394 | Hypothetical protein | *Oryza sativa* | 1413 |

### Example 3: Discovery of a Picrocrocin-forming UGT

A glucosyltransferase enzyme is required to form picrocrocin from hydroxyl-beta-cyclocitral (HBC). This reaction is an aglycon glucosylation as opposed to a glucose-glucose bond-forming reaction, and there are many families of UDP-glucose utilizing glycosyltransferases to screen for this type of activity.

### Sourcing of HBC substrate

HBC was synthesized and the desired compound was purified by chiral column chromatography (GVK, Hyderabad).

### Screening of UGT enzymes

The following UGTs were assayed for picrocrocin formation: *Stevia rebaudiana* 88B1, 76G1, 74G1, 91D2e, 85C2, 73EV12; *Catharanthus roseus* UGT2; and *Arabidopsis thaliana* UGT 75B1, and *Arabidopsis* hybrid enzymes UGT 353 and UGT354 (sequences provided in FIG. 3).

The genes encoding these UGTs were cloned into plasmids utilizing the T7 promoter and transformed into *E. coli* BL21 cells for expression studies. Strains harboring these UGTs were induced with 0.1 mM IPTG and induced cultures were grown at 20°C overnight. Induced cells then were lysed with BugBuster reagent (Novagen) and the clarified lysates were used for the UGT assay.

The UGT assay was performed in 100 µL reactions with 98 µL induced clarified lysates added to HBC as the glucose-acceptor substrate (10 µM final concentration) and UDP-glucose (1 mM final concentration) as the donor. Reactions were performed at 30°C for 3 hours and terminated by addition of 300 µL of water saturated 1-butanol. The samples were extracted three times with 300 µL of water-saturated 1-butanol. The pooled butanol fractions were dried to completion in a Speed-vac and analyzed by LC/MS, using the following method. A Luna-SL C18 column (5 µm, 100 Angstrom) model G1316B (4.6 mm ID) was used for the LC separation, monitoring at 440 nm. A 20 minute separation is performed at 0.8 ml/minute using a gradient from 20-80% acetonitrile with the other solvent being 0.25% formic acid (FA). The LC is coupled to a Q-TOF for MS analysis.

Of these UGTs, UGT85C2 and UGT73EV12 from *Stevia* and the two hybrid *Arabidopsis* enzymes showed formation of picrocrocin from HBC under the conditions assayed. The preliminary analysis showed that the reactions with *Stevia* UGT85C2 partially converted the HBC into a compound with a retention time and mass similar to the picrocrocin standard. HBC peak area was monitored at the retention time of the standard.

The *Stevia* UGT85C2 is co-expressed in the yeast strain that has been shown to produce HBC (see Examples 2 & 6). It is expected that this enzyme will catalyze the same reaction *in vivo* as shown *in vitro,* such that the yeast strain will be capable of producing picrocrocin from glucose.

### Screening UGT collection

A collection of over 170 UGT enzymes with broad ranges of specificity were expressed in *E. coli,* and assayed in a similar way as described above. Three additional UGTs were identified that can perform a glycosylation reaction with HBC to form picrocrocin: *Stevia* UGT73, and two *Arabidopsis* UGT71 hybrid enzymes (see Hansen, et al., Phytochemistry 70 (2009) 473-482 regarding the hybrid enzymes). FIG. 3 provides the nucleotide and amino acid sequences of UGT73 and the UGT71 hybrid enzymes.

### Example 4: Discovery of crocin-forming glyosyltransferase enzymes

Crocin is a derivative of crocetin that has four glucose moieties added to it in successive reactions. The final two glucose molecules are attached to the two primary glucose molecules by β-1,6-bonds, very likely by the action of one glycosyltransferase. UGT enzymes that catalyze the addition of a second glucose are less common than aglycone glycosylase transferases, and are likely be of UGT sub-family 91 or 79. These two subfamilies are the only two known currently to catalyze the formation of 1,2 or 1,6 glucose-glucose bonds.

In an effort to identify genes from *Crocus*, sub-family 79 and 91 UGTs from *Crocus* stigma were identified and isolated, as well as other sub-family 91 UGTs.

### Crocus pyrosequencing

Pyrosequencing data for *Crocus* stigma cDNA was received from MOgene LC (St. Louis, MO, USA). Total transcriptome sequencing was executed using two FLX Titanium plates, and raw sequencing data of total approximately 1100 MB was generated and *de novo* assembly was performed.

After analyzing 66,000 unique contigs of pyrosequenced data, about 10 UGT-like sequences (sub-family 91) were identified by blast analysis against known UGTs. Based on this, gene/allele specific inverse PCR primers were designed to isolate full-length genes from a *Crocus* cDNA library.

Gene- and vector-specific primers were designed based on the pyrosequencing data and used to get the 5'- and 3'-ends of the UGT genes. After successful amplification of the 5' and 3' ends of the UGT sequence with a combination of gene and vector specific primers using proof reading polymerases (e.g., Advantage 2 and KOD polymerases), amplified PCR fragments were gel extracted for downstream processing. PCR amplified fragments were purified using a PCR purification kit and then subsequently were cloned into a TA cloning vector (InstaTA cloning kit, Fermentas), and transformed into *E*. *coli* strain (NEB 10-β Competent cells, New England Biolabs, UK). After qualitative analysis of PCR fragments with gene specific colony-PCR, plasmid DNA samples were sequenced.

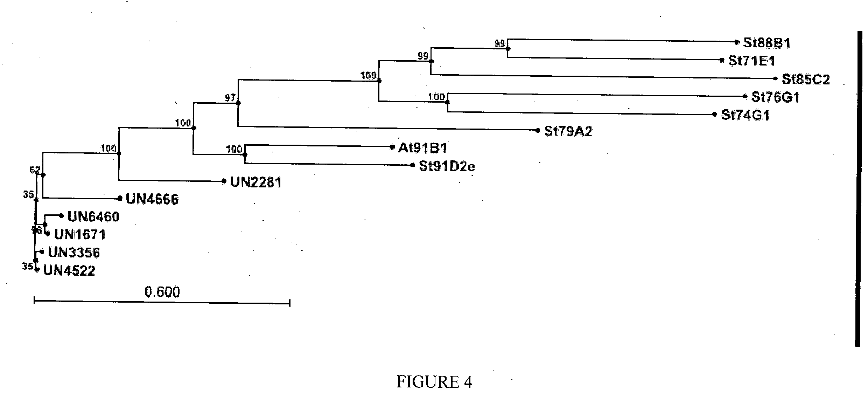

Six full-length UGT Crocus cDNA sequences from sub-family 91 were identified in this manner. The amino acid sequences of all six UGTs (UN1671, UN3356, UN4522, UN4666, UN6460 and UN2281) cluster with known UGT91 sequences (see FIG. 4; FIG. 5 contains the sequence of UN1671, 3356, 4522, 4666, 6460 and 2281). Amongst these six, the UN1671 transcript and UN4522 transcripts were the most highly expressed of the 91 homologs found, based on its abundance in the transcriptome.

The six full-length sequences of UN1671, UN4522, UN4666, UN6460 UN3356 and UN2281 were further amplified with gene specific primers and inserted in plasmid vectors for *E. coli* expression and *in vitro* expression.

The SMART PCR cDNA synthesis approach was utilized for the amplification of the complete sequence of an additional seven UGTs. This approach has the capacity to produce high-quality cDNA from nanogram amounts of total RNA. RACE cDNA was prepared from *Crocus* mRNA that had been purified based on affinity methods that capture the polyadenylated region of the mRNA. Gene specific and allele specific primers are utilized to obtain full length UGT coding regions. The coding regions were transformed in *E. coli* T7 Express *lysY*/*I^{q}* Competent *E. coli* (New England Biolabs, UK) strain harboring the respective UGTs, grown in Luria Broth media containing antibiotic and incubated at 37°C for 16 hrs (shaking at 250 rpm). The cells were inoculated to an OD600 of 0.01 in fresh LB and grown at 30°C until an OD600 of 0.4 to 0.5 is reached. The temperature was lowered to 20°C and cells were induced with 0.1 mM IPTG and incubated for 24 hours. The cells were pelleted at 12,000 rpm for 1 minute at room temperature and lysed in Bug buster reagent (Novagen) as per manufacturer's protocols. Clarified supernatant was used for UGT assays using 10 mM UDP-glucose (final concentration) and 1 mM di-glucosyl ester (final concentration) in reactions incubated at 30°C for 3 hours.

### Screening of in vitro translated enzymes

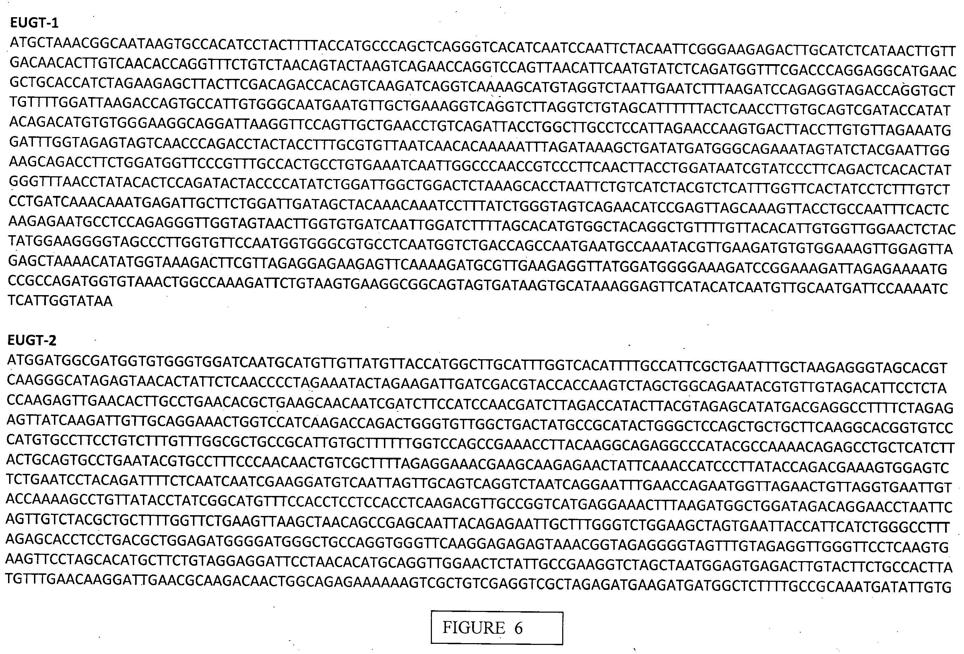

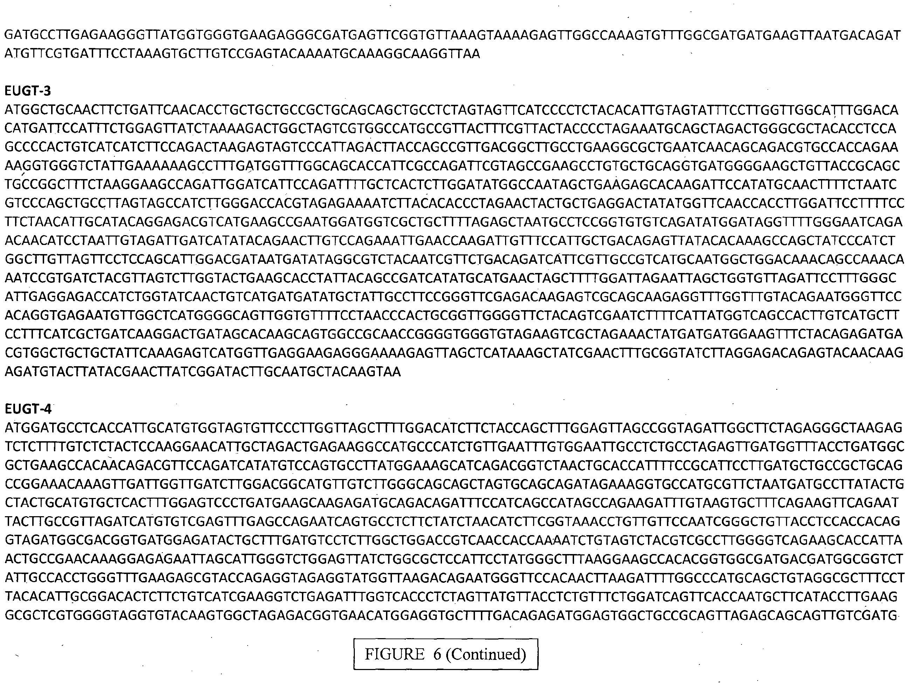

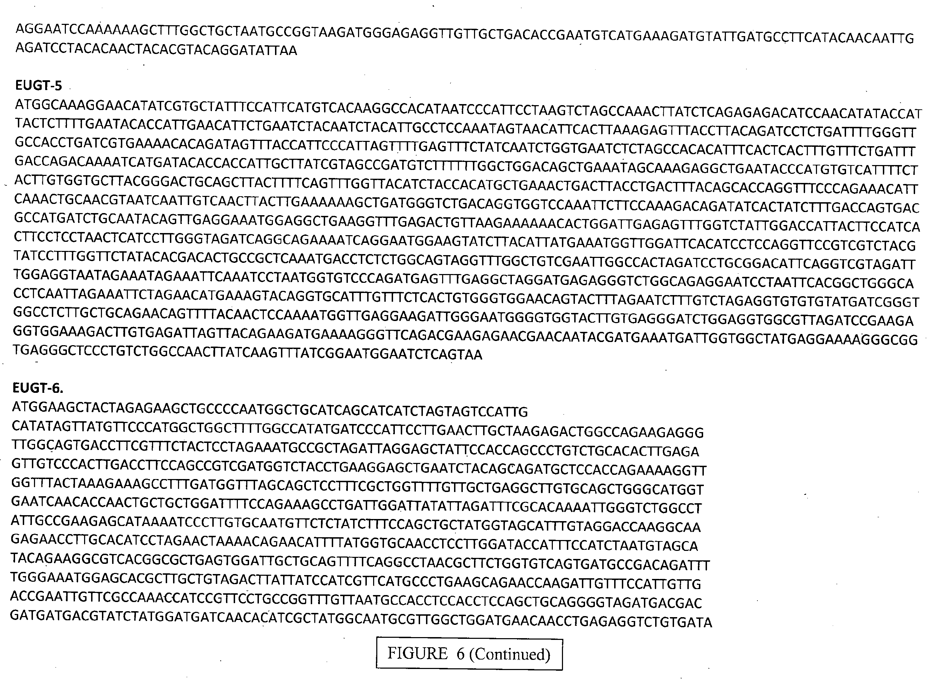

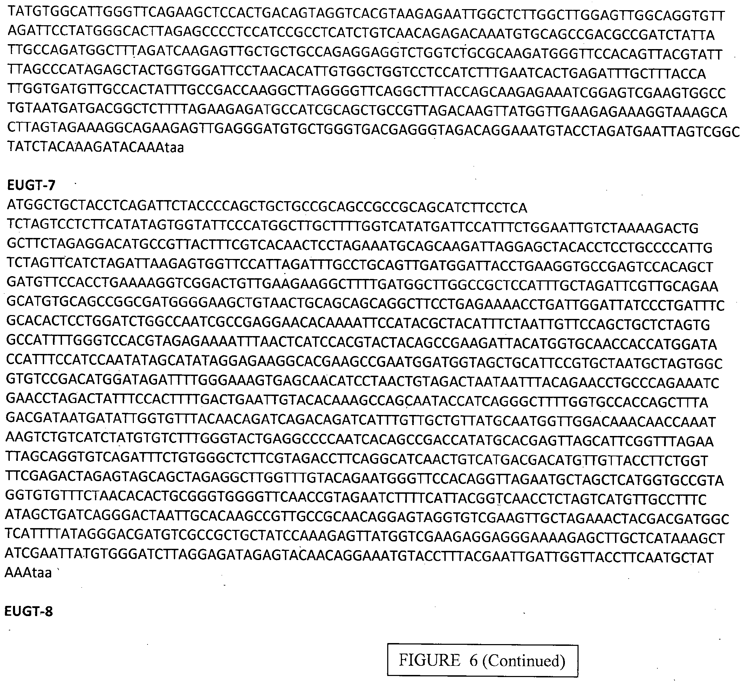

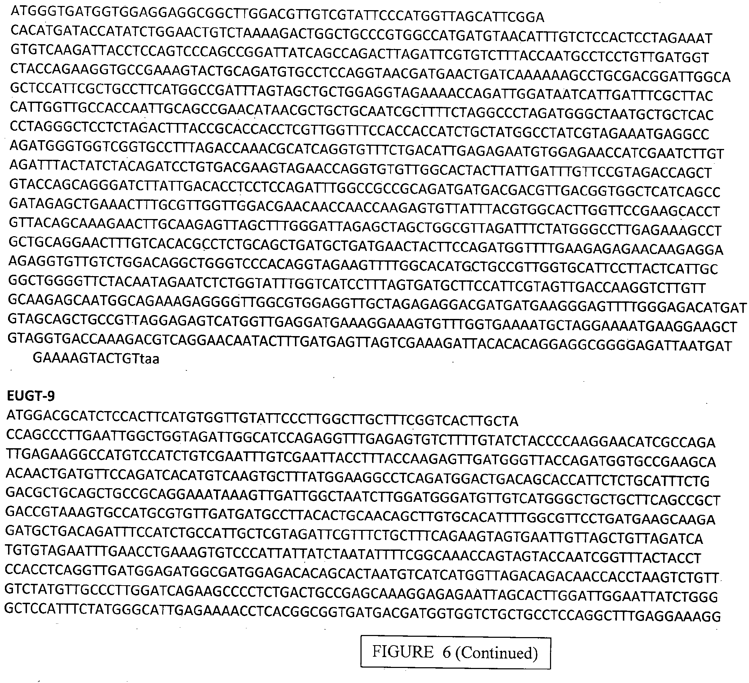

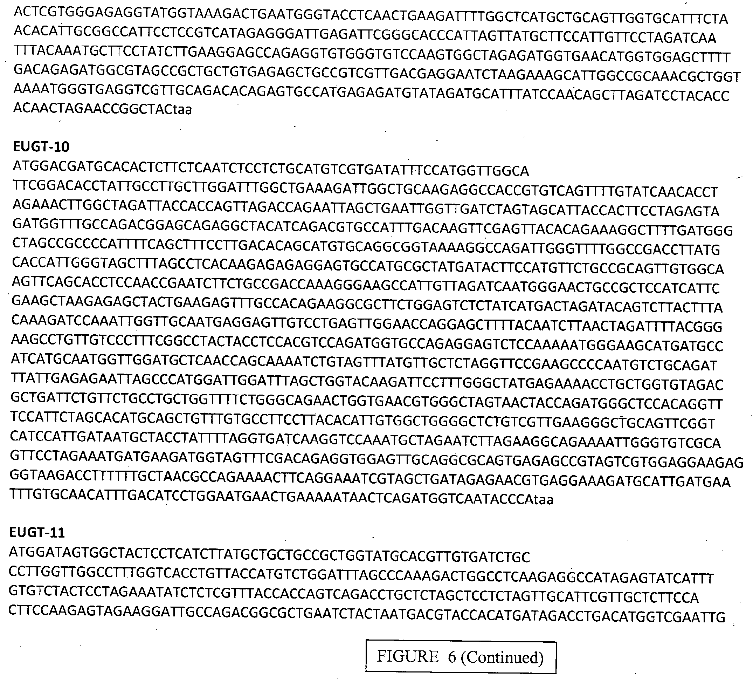

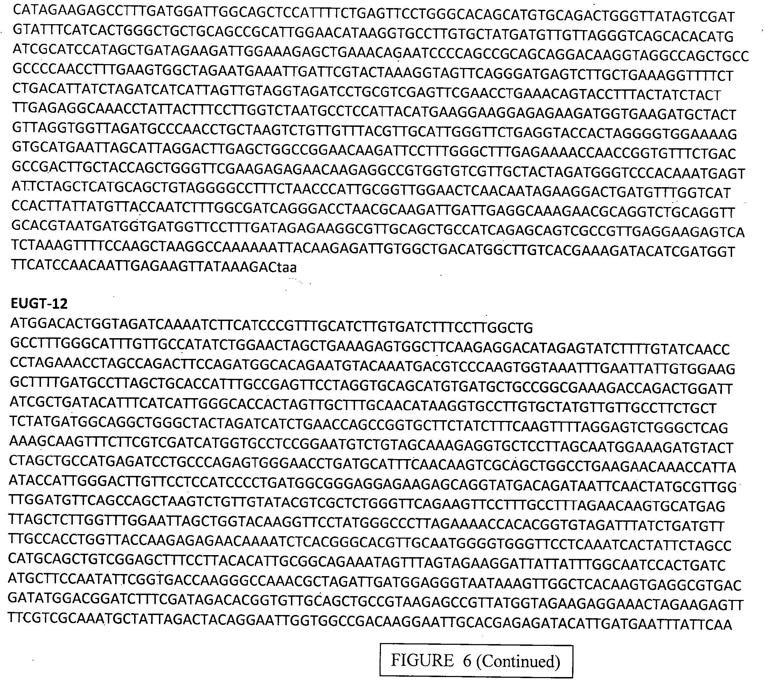

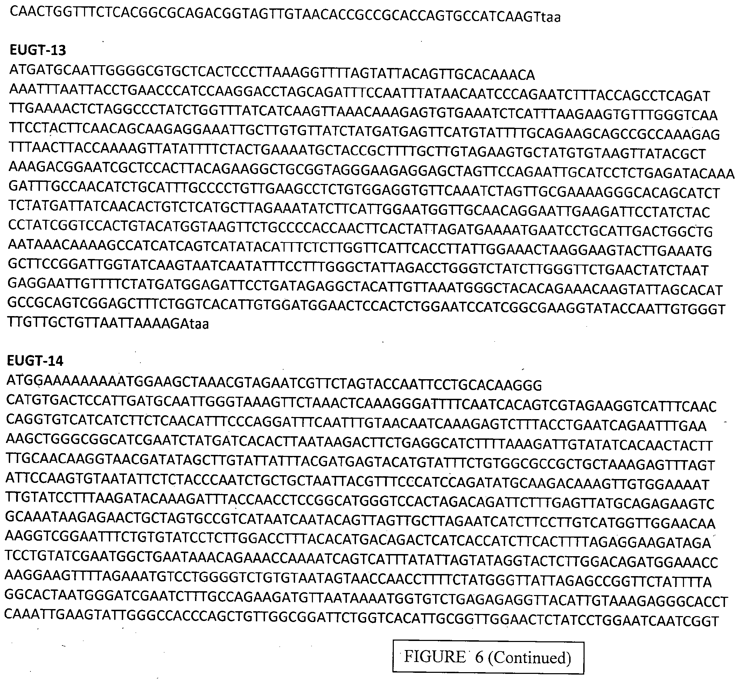

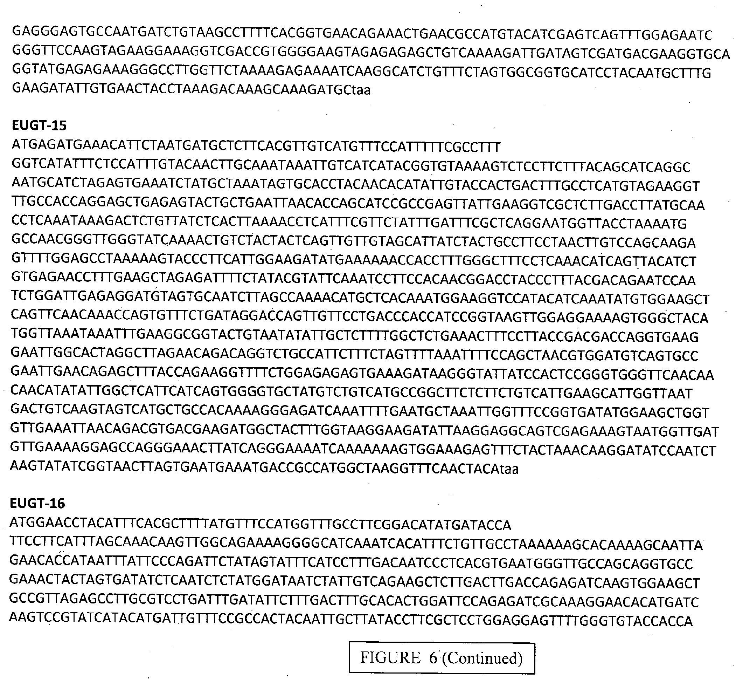

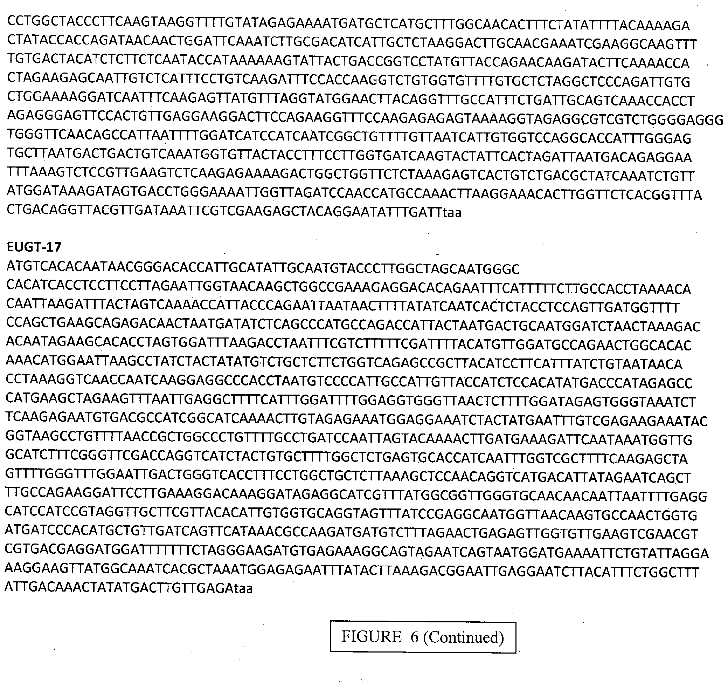

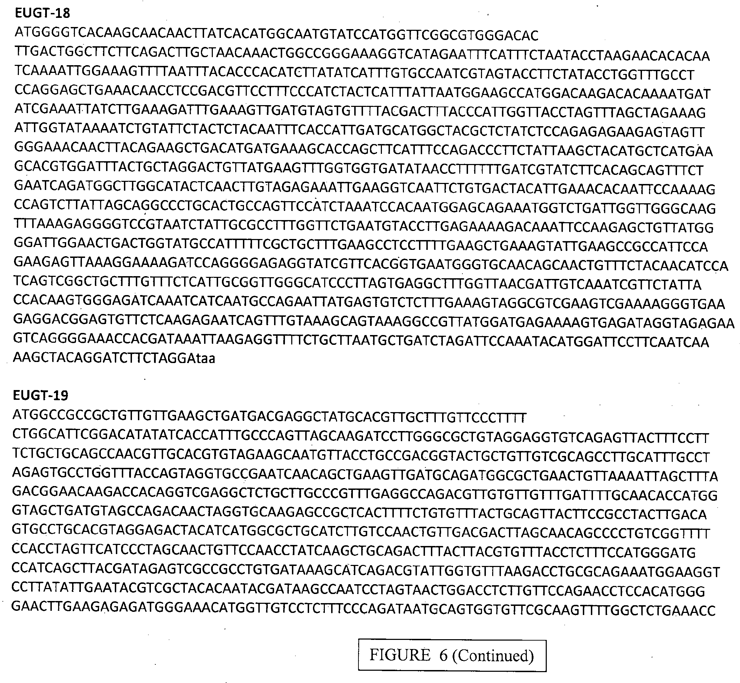

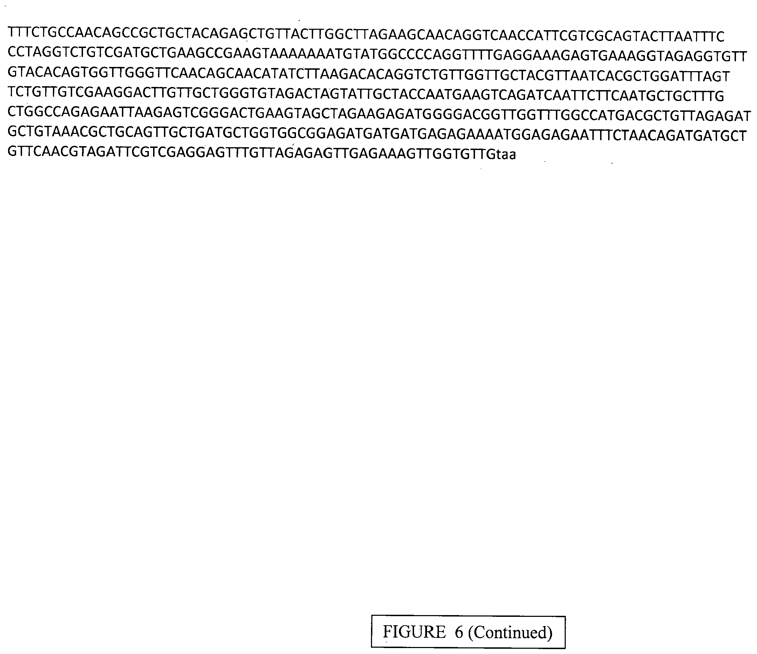

A total of 19 UGT genes (see Table 2) were selected as candidates for conversion of partially mono-glycosylated crocetin esters to crocin due to their homology with other sub-family 79 or 91 UGT sequences. All genes were synthesized with optimization for yeast codon usage (nucleotide sequences in FIG. 6).

**TABLE 2**

| **Code** | **Accession No** | **Gene Name** | **Size (bps)** |
|---|---|---|---|
| EUGT-1 | AY262037 | glucosyltransferase 2, UGTCs2 | 1383 |
| EUGT-2 | AP003270 | putative UDP-glucosyltransferase | 1422 |
| EUGT-3 | AP005171 | putative UDP-glucosyltransferase | 1524 |
| EUGT-4 | AP005643 | putative UDP-glucosyltransferase | 1296 |
| EUGT-5 | A Y290820 | glucosyltransferase , UGTCs3 | 1428 |
| EUGT-6 | AP005259 | putative UDP-glucosyltransferase | 1539 |
| EUGT-7 | AP005171 | putative UDP-glucosyltransferase | 1524 |
| EUGT-8 | XM_470006 | putative UDP-glucoronosyl and UDP-glucosyl | 1452 |
| EUGT-9 | AP005643 | putative UDP-glucosyltransferase | 1296 |
| EUGT-10 | AC133334 | putative UDP-glucoronosyl and UDP-glucosyl transferase | 1419 |
| EUGT-11 | AC133334 | putative UDP-glucoronosyl and UDP-glucosyl transferase | 1389 |
| EUGT-12 | AP004741 | putative UDP-glucosyltransferase | 1338 |
| EUGT-13 | AB012241 | glucosyltransferase-like protein | 1056 |
| EUGT-14 | AL133314 | glucosyltransferase-like protein | 1317 |
| EUGT-15 | Z25802 | UDP rhamnose: anthocyanidin-3-glucoside rhamnosyltransferase | 1416 |
| EUGT-16 | AC004786 | putative flavonol 3-O-glucosyltransferase | 1329 |
| EUGT-17 | AB294391 | glucosyltransferase | 1386 |
| EUGT-18 | AB192314 | glucosyltransferase | 1380 |
| EUGT-19 | NM_ 0010743 94 | Hypothetical protein | 1413 |

| | | | |
|---|---|---|---|
| * Could not be PCR amplified with T7 promoter sequence for *in vitro* translation/expressed for EUGTs # 2, 8 and 11 | | | |

*In vitro* translation was successful for 16 UGTs; the other three UGTs were cloned into an *E. coli* based expression system. The 16 *in vitro* translated UGTs were screened for crocin formation using crocetin gentiobiosylglucosyl ester (crocetin-3G, GVK, India) as the glucose-acceptor substrate and UDP-glucose as the glucose donor. Forty µL of *in vitro* translated protein was used in a 100 µL reaction containing 3 mM final concentration of MgCl₂, 10 µg/mL BSA, 50 µM substrate, and 1 mM UDP-glucose. Reactions were performed at 30°C for 3 hours in 50 mM potassium phosphate buffer pH 7.2 and terminated by adding 300 µL of water saturated 1-butanol. The samples were extracted three times with 300 µL of water-saturated 1-butanol. The pooled butanol fractions were dried completely in a Speed-Vac, resuspended in methanol, and analyzed by an Agilent 1200 HPLC & Q-TOF LC/MS 6520. None of samples tested appeared to produce crocin under the reaction conditions assayed.

### Screening of plant UGT enzymes

Five UGTs from *Stevia* (88B1, 76G1, 74G1, 912D2e, and 85C2) as well as the *Catharanthus roseus* UGT2 and the *Arabidopsis thaliana* UGT 75B1 (see example 3) also were assayed for crocin production.

Among these UGTs, Crocus UGTs UN1671 and UN4522 and the *Stevia* UGT76G1 demonstrated the ability to glycosylate crocetin-3G. Preliminary analysis by LC-MS showed the appearance of a product molecule with the same molecular mass of crocin. As UGTs of sub-family 76 typically makes a 1,3 bond between two glucose moieties, the type of glucose-glucose linkage is verified by NMR to determine whether crocin or a crocin analog has been produced.

### Example 5: Cloning of Crocus UGT2 for crocetin glucosyl ester formation

*Crocus* UGT2 (CsUGT2, GenBank Accession Number: AY262037.1) is thought to catalyze the two primary glucosylations of the crocetin at the carboxylate positions, resulting in crocetin mono- and di-glucosyl esters. The CsUGT2 was cloned, with and without a poly-histidine tag fusion, into a bacterial expression vector using the T7 promoter. The gene also was cloned into a yeast expression construct using the strong constitutive GPD1 promoter. A gene for optimized yeast expression was utilized for the cloning. FIG. 7 provides the nucleotide and amino acid sequences of the CsUGT2, as well as the codon-optimized nucleotide sequence.

The transformed XJa (DE3) autolysis *E. coli K* strains are induced with IPTG according to manufacturer's protocols (Zymo research, CA 92614, U.S.A). The transformed *Saccharomyces cerevisiae* cells (Strain DSY5, Dualsystems Biotech, Switzerland) are grown in SC dropout media containing 2% glucose, pH 5.8. Single colonies of DSY5 strain harboring the CsUGT2 gene are inoculated in SC glucose media and incubated at 30°C at 250 rpm overnight. The yeast cells are re-inoculated in fresh media to an equivalent of 1.0 OD600 in fresh SC broth and incubated for an additional 72 hours. Cells are then pelleted and lysed using YeastBuster™ Protein Extraction Reagent (Merck, India). The cell-free extracts are assayed for crocetin glycosylation activity using 10 mM UDP-glucose (final concentration), 1mM Crocetin (final concentration) purchased from Chromadex (US), and incubated at 30°C for 3 hours. Analysis is done on crude reaction mixtures and the presence of mono and di-glucosyl esters are observed based on their masses, using mass spectrometry as per the reference J. Mass. Spectrom. 2009, 44, 1661-1667

### Example 6: Yeast producing crocetin

A functional biosynthesis pathway for production of crocetin was developed as follows. The engineered yeast strain (EYS886) described in Example 1, producing β-carotene, was used for for engineering the saffron biosynthesis pathway. The co-expression of the *C*. *sativus* zeaxanthin cleavage oxygenase (ZCO, also known as zeaxanthin cleavage dioxygenase or ZCD) and *Xanthophyllomyces dendrorhous* carotene hydroxylase (CH) CH-2 genes resulted in production of crocetin as evidenced by LC and MS analysis. A heterologous gene was not provided for the conversion of the crocetin dialdehyde to crocetin; this activity must occur natively in the *S*. *cerevisiae* cells.

The high copy number pRS416 *E.coli*/*yeast* shuttle vectors were utilized for expression of several combinations of gene analogs of carotene hydroxylase ("CH") and zeaxanthin cleavage oxygenase ("ZCO") sourced as described in Table 3 (FIG. 8 contains the optimized DNA sequences). The ZCO genes were expressed under the control of the TEF promoter; the CH genes were expressed using the GPD promoter. The following combinations were tested: CH2/ZCO1, CH3/ZCO2, and CH6/ZCO4.

**Table 3 Sources of CH and ZCO genes**

| | | |
|---|---|---|
| CH2 | *Xanthophyllomyces dendrorhous* (Fungi) | β-carotene 3-hydroxylase (crtS) |
| CH3 | *Arabidopsis thaliana* (plant) | β-ring hydroxylase (CHY2) |
| CH6 | *Adonis aestivalis* | β-carotene hydroxylase |
| ZCO1 | *Crocus sativus* | zeaxanthin cleavage dioxygenase (CsZCO) |
| ZCO2 | *Crocus sativus* | carotenoid 9,10(9',10')-cleavage dioxygenase (CsCCD) |
| ZCO4 | *Chrysanthemum x morifolium* | Carotenoid Cleavage Dioxygenase (CmCCD4a) |

Plasmids containing the ZCO/CH6 combinations were transformed into the β-carotene producing strain as per manufacturer's protocols (Frozen-EZ Yeast Transformation II Kit,™ Zymo research, Switzerland). The transformants were plated on SC Ura- plates (pH 5.8) containing 2% glucose and incubated at 30°C for 3 days.

Positive yeast clones were grown in liquid SC Ura-media containing glucose at 30°C, aerated at 200 rpm, in a shaking incubator overnight.

Cultures were concentrated by centrifugation, and resuspended in fresh SC Ura-media to an OD equivalent to 1.2. The cells were further incubated at 30°C at 200 rpm for an additional 72 hours. The cells were then pelleted and extracts were prepared for analysis. The pellets were washed with cold PBS buffer (10mM; pH7.2) twice, suspended in 2ml of methanol:PBS buffer (3:1) and stored at -18 °C overnight. This mixture was thawed and centrifuged at 10,000 rpm for 3 minutes and the pellets were re-extracted, using a vortex mixer, with 3 ml of chloroform:methanol (1:2). This mixture was centrifuged at 10,000 rpm for 2 minutes and the supernatant was injected for analysis by HPLC. In a similar manner the supernatant was extracted with chloroform, methanol, and water in the order given and analyzed by HPLC.

### Analysis

Cell extracts were analyzed using a C18 Discovery HS HPLC column with a linear methanol gradient of 60% to 100% in 1% acetic acid and water over a 40 minute period at 1 ml/min. A Shimadzu preparative LC 8A system was utilized with a Shimadzu SPD M20A Photo Diode Array detector with primary analysis at 440 nm absorbance.

The analysis of one of the recombinant strains containing the *C*. *sativus* ZCO1 (GenBank accession number AJ489276, GenBank protein ID CAD33262.1) *and X. dendrorhous* CH-2 revealed the production of new compounds eluting at times comparable with standards of crocetin and crocetin dialdehyde. The intracellular metabolites produced by this yeast strain were further subjected to GC-MS analysis and the masses of crocetin and crocetin dialdehyde were confirmed.

It is expected that other combinations of ZCO and CH also would be functional under conditions appropriate for soluble protein expression.

These data demonstrate that yeast is capable of making crocetin dialdehyde from glucose, and that yeast has an enzymatic activity which can oxidize at least some crocetin dialdehyde to crocetin. Additionally, since HBC is a byproduct of the ZCO reaction, the yeast is also capable of producing HBC. With the addition of the UGTs and the CsUGT2 described above, it is expected that the yeast also will produce picrocrocin and crocin.

### Example 7: Discovery of glycosyltransferase enzymes forming Crocetin esters

It has been proposed that crocetin is enzymatically glucosylated by a multi-step pathway involving two distinct UGTs. One UGT would catalyze the addition of glucose moieties to the terminal carboxyl ends of crocetin with formation of the monoglucosyl-and diglucosyl-esters. The other UGT would transfer glucose moieties to glucosyl groups forming crocetin monogentiobiosyl- and digentiobiosylesters.

The following UGTs were screened for the formation of cocetin esters like mono, di or gentiobiosyl molecules from crocetin: *Stevia rebaudiana* (88B1, 76G1, 74G1, 912D2e, and 85C2, UGT73) and two *Arabidopsis* UGT71 hybrid enzymes (71C125571C2 and 71C125571E1).

The genes encoding these UGTs were cloned into plasmids under the T7 promoter and transformed into *E*. *coli* BL21 (Autolysis: XJb(DE3), Zymoresearch) cells for expression studies. Strains harboring these UGTs were induced with 0.1 mM IPTG and induced cultures were grown at 20°C overnight. Induced cells then were lysed by freeze and thaw method.

The UGT assay was performed in 100 µL reactions with 98 µL induced clarified lysates incubated with Crocetin as the glucose-acceptor substrate (10 µM final concentration) and UDP-glucose (1 mM final concentration) as the donor. Reactions were performed at 30°C for 3 hours and terminated by addition of 300 µL of water saturated 1-butanol. The samples were extracted three times with 300 µL of water-saturated 1-butanol. The pooled butanol fractions were dried to completion in a Speed-vac and analyzed by LC/MS, using the following method. Instrument: Agilent 1200 HPLC & Q-TOF LC/MS 6520, Column: c18 reverse Luna, 4µm, 4.6×150mm,Injected volume : 20µl, Mobile phase : Acetonitrile (B): Water (A) (0.1% HCOOH) in binary, flow rate: 0.8 ml/min, run time: 20min, detection: 440nm, gradient: 20% B for 5 min, 80% B for 15 min, 80% B in 20 min, Ion source -Dual ESI, Acquisition Mode- MS, Mass Range-100-1500, Mode-Negative modes

Among these, three UGTs (76G1 from Stevia, and the two *Arabidopsis* UGT71 hybrid enzymes) catalyzed the glucosylation of crocetin to form mono and di glucosyl esters. The two *Arabidopsis* UGT71 hybrid enzymes (71C125571C2 and 71C125571E1) also demonstrated the ability to form Crocetin gentibiosyl ester. Preliminary analysis by LC-MS showed the appearance of product molecules with the same molecular mass of mono, di and gentibiosyl esters.

### Example 8: Discovery of crocetin mono and di glucosyl ester forming glycosyltransferase from Crocus sativus

The pyrosequencing data of Example 4 also revealed a variant Crocus UGT, Cs VrUGT2. FIG. 9 contains the amino acid sequence of Cs VrUGT2. The sequence of the variant UGT was compared to the Crocus UGT2 (CsUGT2, GenBank Accession No.: AY262037.1) using BLAST. FIG 10 contains the alignment of CsUGT2 and variant Cs VrUGT2 from *Crocus sativus,* as well as the amino acid sequence of each polypeptide. Based on the BLAST analysis, gene/allele specific inverse PCR primers were designed to isolate full-length genes from a Crocus cDNA library.

A codon optimized nucleotide sequence encoding Cs VrUGT2 was cloned into plasmids under the T7 promoter and transformed into *E. coli* BL21 (Autolysis: XJb(DE3), Zymoresearch) cells for expression studies. A strain harboring the Cs VrUGT2 was induced with 0.1 mM IPTG and the induced cultures were grown at 20°C overnight. Induced cells then were lysed by freezing and thawing.

The UGT assay was performed in 100 µL reactions with 98 µL of clarified lysates from induced cultures, incubated with Crocetin as the glucose-acceptor substrate (10 µM final concentration) and UDP-glucose (1 mM final concentration) as the donor. Reactions were performed at 30°C for 3 hours and terminated by addition of 300 µL of water saturated 1-butanol. The samples were extracted three times with 300 µL of water-saturated 1-butanol and the fractions pooled. The pooled butanol fractions were dried to completion in a Speed-vac and analyzed by LC/MS, using the following method. Instrument: Agilent 1200 HPLC & Q-TOF LC/MS 6520, Column: c18 reverse Luna, 4µm, 4.6×150mm, Injected volume: 20µl, Mobile phase: Acetonitrile (B): Water (A) (0.1%HCOOH) in binary, flow rate: 0.8 ml/min, run time: 20min, detection: 440nm, gradient: 20% B for 5 min, 80% B for 15 min, 80% B in 20 min, Ion source-Dual ESI, Acquisition Mode- MS, Mass Range-100-1500, Mode- Negative modes

Preliminary analysis by LC-MS showed the appearance of product molecules with the same molecular mass of mono and di glucosyl esters.

### Example 9: Discovery of endogenous yeast aldehyde dehydrogenases that can covert crocetin dialdehyde to crocetin

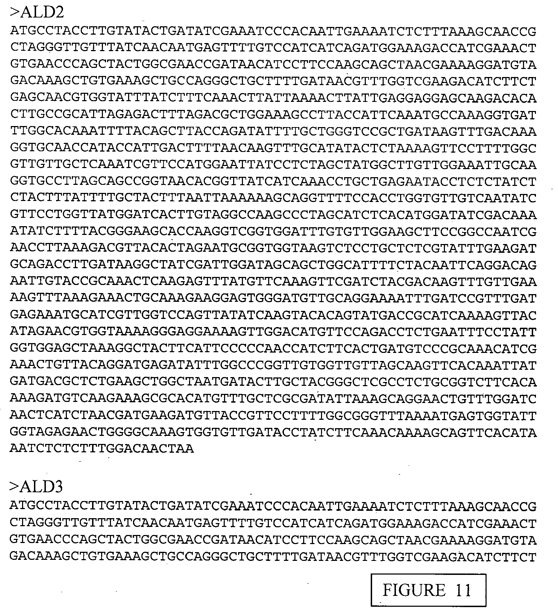

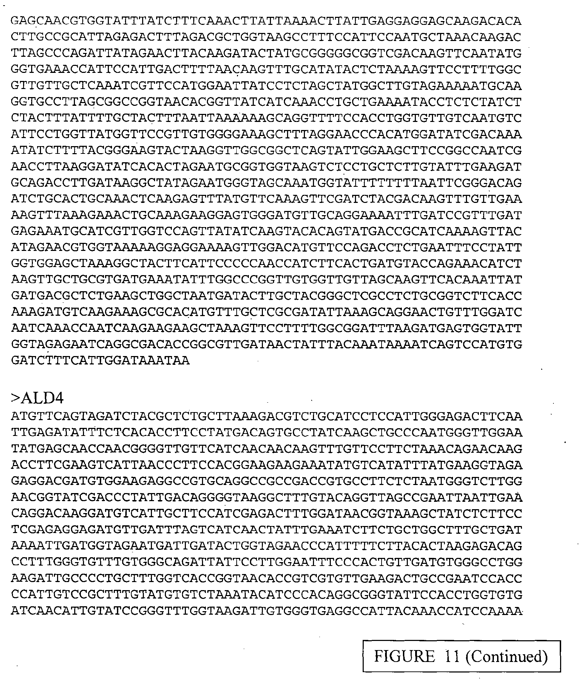

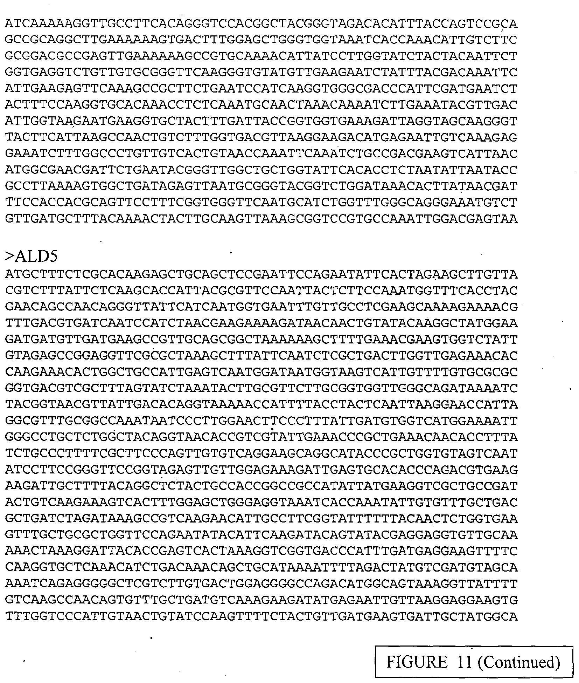

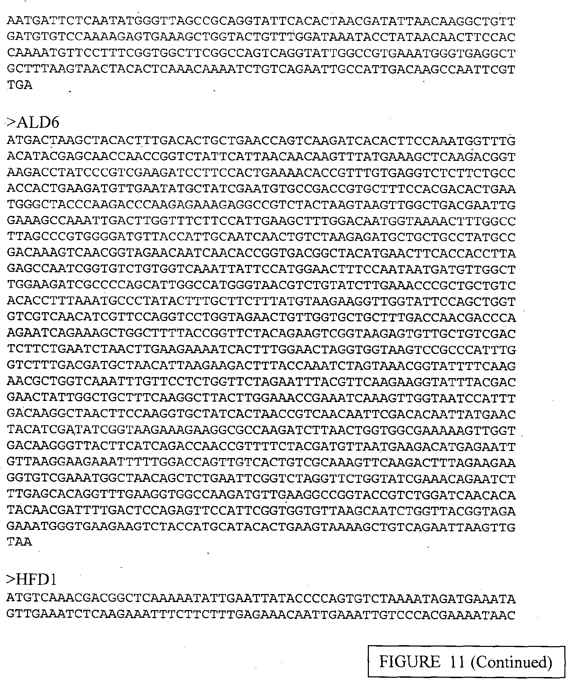

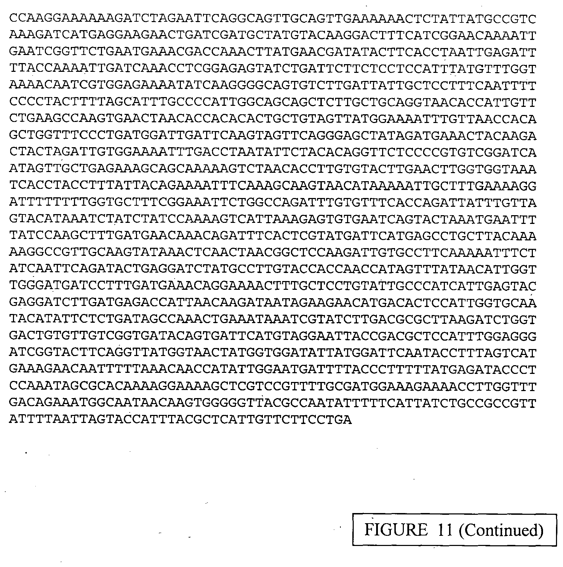

The color of saffron is mainly due to the carotenoid glycosides derived from the sequential glycosylation of crocetin. One of the key steps in the saffron bio-synthetic pathway is the oxidation of crocetin dialdehyde to crocetin. The ability of endogenous aldehyde dehydrogenases in *Saccharomyces cerevisiae* to effect this conversion was tested. The yeast genome has five known aldehyde dehydrogenase coding genes (ALD2 through ALD6) as well as an additional gene, HFD1, which is predicted to be an aldehyde dehydrogenase. See FIG. 11 for the nucleotide sequences encoding ALD2, ALD3, ALD4, ALD5, ALD6, and HFD1 from reference strain S288C (SEQ ID NOs. 67-72). The sequences are for the reference strain S288C. There can be slight changes in the gene sequences in the strain that has been used. Cell free extracts were prepared from yeast cultures grown overnight and then disrupted by mechanical lysis. The lysates were clarified and tested for their ability to convert crocetin dialdehyde to crocetin in *in vitro* reactions carried out as set forth in Table 4. A negative control without any whole cell extract also was included. The reactions were performed at 25°C for 60 minutes then stopped by adding three volumes (1500 ml) of water saturated butanol.

**Table 4**

| **Component** | **Final concentration** | **Amount per reaction** |
|---|---|---|
| 1M Tris-HCl pH7.5 | 100mM | 50 µl |
| 1M KCl | 100mM | 50 µl |
| 0.5M MgCl₂ | 3.75mM | 3.75 µl |
| 1M 2-mercaptoethanol | 10mM | 5 µl |
| 10 mM Crocetin dialdehyde | 200µM | 10 µl |
| 20 mM β-NAD | 0.67mM | 16.7 µl |
| Cell free extract | | 50 µl |
| Water | | 314.55 µl |
| **Total** | | **500µl** |

The organic phase was separated by centrifugation and subjected to vacuum drying after which they were analyzed by high performance liquid chromatography coupled with mass spectroscopy (LC-MS). An Agilent 1200 HPLC & Q-TOF LC/MS 6520 was used, with a Luna C18 5µm column (4.6 x 150 m) equipped with 5 micron guard column. The mobile phase was Acetonitrile (B) (0.1% formic acid (HCOOH)): H₂O (A) (0.1% HCOOH), with a flow rate of 0.8 ml/min. Run time was typically 15 min with 1 min post run.

| **Time** | **Solvent Ratio B** |
|---|---|
| 4 | 70 |
| 10 | 80 |
| 12 | 90 |
| 15 | 90 |

MS parameters included the following: ESI as an ion source, dual ESI acquisition mode; 100-450 Da mass range; +/- ve (fast polar switching) mode.

The yeast endogenous aldehyde dehydrogenase(s) were able to convert crocetin dialdehyde to crocetin as demonstrated by the LC-MS results.

### OTHER EMBODIMENTS

It is to be understood that while the invention has been described in conjunction with the detailed description thereof, the foregoing description is intended to illustrate and not limit the scope of the invention, which is defined by the scope of the appended claims. Other aspects, advantages, and modifications are within the scope of the following claims.

## Claims

1. A recombinant, carotenoid producing host cell, comprising:
(a) an exogenous nucleic acid encoding a zeaxanthin cleavage dioxygenase (ZCD), wherein said ZCD is optionally a *Crocus sativus* ZCD; and
(b) an exogenous nucleic acid encoding a β-carotene hydroxylase (CH),
wherein the host cell produces detectable amounts of any one or more of crocetin dialdehyde, crocetin, or hydroxyl-β-cyclocitral (HBC).

2. The host cell of claim 1, further comprising endogenous genes encoding a geranylgeranyl diphosphate synthase (GGPPS), a phytoene synthase, a phytoene dehydrogenase, and a β-carotene synthase.

3. The host cell of claim 1 or 2, further comprising at least one exogenous nucleic acid encoding a GGPPS, a phytoene synthase, a phytoene dehydrogenase, and a β-carotene synthase.

4. The host cell of any one of claims 1 to 3, wherein said host cell further comprises an endogenous gene encoding a β-carotene hydroxylase (CH) or an aldehyde dehydrogenase (ALD), or an exogenous nucleic acid encoding an ALD.

5. The host cell of any one of claims 1 to 4, said host cell further comprising an exogenous nucleic acid encoding an aglycone O-glycosyl uridine 5'-diphospho (UDP) glycosyl transferase (O-glycosyl UGT).

6. The host of claim 5, wherein said aglycone O-glycosyl UGT is UGT85C2 having an amino acid sequence set forth in SEQ ID NO:10 or UGT73 having 80% or greater sequence identity to the amino acid sequence set forth in SEQ ID NO:12.

7. The host cell of claim 5, wherein said host cell produces detectable amounts of picrocrocin or crocin.

8. The host cell of any one of claims 1 to 7, said host cell further comprising a CsVrUGT2 having 80% or greater sequence identity to the amino acid sequence set forth in SEQ ID NO:66.

9. The host cell of claim 8, wherein said host cell produces a detectable amount of a crocetin monoglucoside, a crocetin diglucoside, crocin or a crocetin ester.

10. The host cell of any one of claims 1 to 9, wherein the host cell further comprises;
an exogenous nucleic acid encoding UGT76G1, which may optionally have 80% or greater sequence identity to the amino acid sequence set forth in SEQ ID NO: 4;
an exogenous nucleic acid encoding 71C125571C2, which may optionally have 80% or greater sequence identity to the amino acid sequence set forth in SEQ ID NO: 18; or
an exogenous nucleic acid encoding 71C125571E1, which may optionally have 80% or greater sequence identity to the amino acid sequence set forth in SEQ ID NO: 20.

11. The host cell of claim 9, wherein the host cell produces a detectable amount of crocetin gentibiosyl ester, crocin or crocetin mono and di glucosyl esters.

12. The host cell of any one of claims 1 to 11, wherein the host cell further comprises a gene encoding UN1761 having 80% or greater sequence identity to the amino acid sequence set forth in SEQ ID NO: 29, or a gene encoding UN4522 having 80% or greater sequence identity to the amino acid sequence set forth in SEQ ID NO: 31.

13. The host cell of any one of claims 1 to 12, wherein the host cell is a microorganism or a plant cell.

14. The host cell of claim 13, wherein the microorganism is a yeast, a Saccharomycete or *Escherichia coli*.

15. The host cell of claim 14, wherein the yeast is an oleaginous yeast.

16. The host cell of claim 14, wherein Saccharomycete is a *Saccharomyces cerevisiae.*

17. The host cell of claim 13, wherein the plant cell is *Crocus sativus.*

## Patentansprüche

1. Rekombinante, Carotinoid produzierende Wirtszelle, die Folgendes umfasst:
(a) eine exogene Nukleinsäure, die eine Zeaxanthin-Spaltungs-Dioxygenase (ZCD) kodiert, wobei die ZCD optional eine *Crocus-sativus*-ZCD ist; und
(b) eine exogene Nukleinsäure, die eine β-Carotin-Hydroxylase (CH) kodiert,
wobei die Wirtszelle nachweisbare Mengen von einem oder mehreren von Crocetindialdehyd, Crocetin oder Hydroxyl-β-cyclocitral (HBC) produziert.

2. Wirtszelle nach Anspruch 1, die weiter endogene Gene umfasst, die eine Geranylgeranyldiphosphat-Synthase (GGPPS), eine Phytoen-Synthase, eine Phytoen-Dehydrogenase und eine β-Carotin-Synthase kodieren.

3. Wirtszelle nach Anspruch 1 oder 2, die weiter mindestens eine exogene Nukleinsäure umfasst, die eine GGPPS, eine Phytoen-Synthase, eine Phytoen-Dehydrogenase und eine β-Carotin-Synthase kodiert.

4. Wirtszelle nach einem der Ansprüche 1 bis 3, wobei die Wirtszelle weiter ein endogenes Gen, das eine β-Carotin-Hydroxylase (CH) oder eine Aldehyddehydrogenase (ALD) kodiert, oder eine exogene Nukleinsäure, die eine ALD kodiert, umfasst.

5. Wirtszelle nach einem der Ansprüche 1 bis 4, wobei die Wirtszelle weiter eine exogene Nukleinsäure umfasst, die eine Aglycon-O-Glycosyluridin-5'-diphospho(UDP)-Glycosyltransferase (O-Glycosyl-UGT) kodiert.

6. Wirt nach Anspruch 5, wobei die Aglycon-O-Glycosyl-UGT Folgendes ist: UGT85C2, die eine Aminosäuresequenz aufweist, die in SEQ.-ID Nr. 10 dargelegt ist, oder UGT73, die 80 % oder mehr Sequenzidentität mit der Aminosäuresequenz, die in SEQ.-ID Nr. 12 dargelegt ist, aufweist.

7. Wirtszelle nach Anspruch 5, wobei die Wirtszelle nachweisbare Mengen von Picrocrocin oder Crocin produziert.

8. Wirtszelle nach einem der Ansprüche 1 bis 7, wobei die Wirtszelle weiter eine CsVrUGT2 umfasst, die 80 % oder mehr Sequenzidentität mit der Aminosäuresequenz, die in SEQ.-ID Nr. 66 dargelegt ist, aufweist.

9. Wirtszelle nach Anspruch 8, wobei die Wirtszelle eine nachweisbare Menge eines Crocetinmonoglucosids, eines Crocetindiglucosids, von Crocin oder eines Crocetinesters produziert.

10. Wirtszelle nach einem der Ansprüche 1 bis 9, wobei die Wirtszelle weiter Folgendes umfasst:
eine exogene Nukleinsäure, die UGT76G1 kodiert, die optional 80 % oder mehr Sequenzidentität mit der Aminosäuresequenz, die in SEQ.-ID Nr. 4 dargelegt ist, aufweisen kann;
eine exogene Nukleinsäure, die 71C125571C2 kodiert, die optional 80 % oder mehr Sequenzidentität mit der Aminosäuresequenz, die in SEQ.-ID Nr. 18 dargelegt ist, aufweisen kann; oder
eine exogene Nukleinsäure, die 71C125571E1 kodiert, die optional 80 % oder mehr Sequenzidentität mit der Aminosäuresequenz, die in SEQ.-ID Nr. 20 dargelegt ist, aufweisen kann.

11. Wirtszelle nach Anspruch 9, wobei die Wirtszelle eine nachweisbare Menge von Crocetingentibiosylester, Crocin oder Crocetinmono- und -diglucosylester produziert.

12. Wirtszelle nach einem der Ansprüche 1 bis 11, wobei die Wirtszelle weiter Folgendes umfasst: ein Gen, das UN1761 kodiert, die 80 % oder mehr Sequenzidentität mit der Aminosäuresequenz, die in SEQ.-ID Nr. 29 dargelegt ist, aufweist, oder ein Gen, das UN4522 kodiert, die 80 % oder mehr Sequenzidentität mit der Aminosäuresequenz, die in SEQ.-ID Nr. 31 dargelegt ist, aufweist.

13. Wirtszelle nach einem der Ansprüche 1 bis 12, wobei die Wirtszelle ein Mikroorganismus oder eine Pflanzenzelle ist.

14. Wirtszelle nach Anspruch 13, wobei der Mikroorganismus eine Hefe, ein Saccharomycet oder *Escherichia coli* ist.

15. Wirtszelle nach Anspruch 14, wobei die Hefe eine oleogene Hefe ist.

16. Wirtszelle nach Anspruch 14, wobei Saccharomycet eine *Saccharomyces cerevisiae* ist.

17. Wirtszelle nach Anspruch 13, wobei die Pflanzenzelle *Crocus sativus* ist.

## Revendications

1. Cellule hôte recombinée productrice de caroténoïdes, comprenant :
(a) un acide nucléique exogène codant pour une dioxygénase de clivage de la zéaxanthine (ZCD), où ladite ZCD est éventuellement une ZCD de *Crocus sativus* ; et
(b) un acide nucléique exogène codant pour une β-carotène hydroxylase (CH),
où la cellule hôte produit des quantités détectables d'un ou plusieurs quelconques parmi le dialdéhyde de crocétine, la crocétine, ou l'hydroxyl-β-cyclocitral (HBC).

2. Cellule hôte selon la revendication 1, comprenant en outre des gènes endogènes codant pour une géranylgéranyl diphosphate synthase (GGPPS), une phytoène synthase, une phytoène déshydrogénase, et une β-carotène synthase.

3. Cellule hôte selon la revendication 1 ou 2, comprenant en outre au moins un acide nucléique exogène codant pour une GGPPS, une phytoène synthase, une phytoène déshydrogénase, et une β-carotène synthase.

4. Cellule hôte selon l'une quelconque des revendications 1 à 3, où ladite cellule hôte comprend en outre un gène endogène codant pour une β-carotène hydroxylase (CH), ou une aldéhyde déshydrogénase (ALD), ou un acide nucléique exogène codant pour une ALD.

5. Cellule hôte selon l'une quelconque des revendications 1 à 4, ladite cellule hôte comprenant en outre un acide nucléique exogène codant pour une aglycone O-glycosyl uridine 5'-diphospho (UDP) glycosyl transférase (O-glycosyl UGT).

6. Hôte selon la revendication 5, où ladite aglycone O-glycosyl UGT est UGT85C2 ayant une séquence d'acides aminés exposée dans SEQ ID n° 10 ou UGT73 ayant une identité de séquence de 80% ou plus par rapport à la séquence d'acides aminés exposée dans SEQ ID n° 12.

7. Cellule hôte selon la revendication 5, où ladite cellule hôte produit des quantités détectables de picrocrocine ou de crocine.

8. Cellule hôte selon l'une quelconque des revendications 1 à 7, ladite cellule hôte comprenant en outre CsVrUGT2 ayant une identité de séquence de 80% ou plus par rapport à la séquence d'acides aminés exposée dans SEQ ID n° 66.

9. Cellule hôte selon la revendication 8, où ladite cellule hôte produit une quantité détectable d'un monoglucoside de crocétine, d'un diglucoside de crocétine, de crocine ou d'un ester de crocétine.

10. Cellule hôte selon l'une quelconque des revendications 1 à 9, où ladite cellule hôte comprend en outre
un acide nucléique exogène codant pour UGT76G1, qui peut éventuellement avoir une identité de séquence de 80% ou plus par rapport à la séquence d'acides aminés exposée dans SEQ ID n° 4 ;
un acide nucléique exogène codant pour 71C125571C2, qui peut éventuellement avoir une identité de séquence de 80% ou plus par rapport à la séquence d'acides aminés exposée dans SEQ ID n° 18 ; ou
un acide nucléique exogène codant pour 71C125571E1, qui peut éventuellement avoir une identité de séquence de 80% ou plus par rapport à la séquence d'acides aminés exposée dans SEQ ID n° 20.

11. Cellule hôte selon la revendication 9, où la cellule hôte produit une quantité détectable d'un gentibiosyl ester de crocétine, de crocine ou de mono- et diglucosyl esters de crocétine.

12. Cellule hôte selon l'une quelconque des revendications 1 à 11, où la cellule hôte comprend en outre un gène codant pour UN1761 ayant une identité de séquence de 80% ou plus par rapport à la séquence d'acides aminés exposée dans SEQ ID n° 29, ou un gène codant pour UN4522 ayant une identité de séquence de 80% ou plus par rapport à la séquence d'acides aminés exposée dans SEQ ID n° 31.

13. Cellule hôte selon l'une quelconque des revendications 1 à 12, où la cellule hôte est un microorganisme ou une cellule végétale.

14. Cellule hôte selon la revendication 13, où le microorganisme est une levure, un Saccharomycète ou *Escherichia coli.*

15. Cellule hôte selon la revendication 14, où la levure est une levure oléagineuse.

16. Cellule hôte selon la revendication 14, où le Saccharomycète est *Saccharomyces cerevisiae.*

17. Cellule hôte selon la revendication 13, où la cellule végétale est *Crocus sativus.*
